# EUROPEAN PATENT APPLICATION

(11) **EP 3 421 607 A1**
(43) Date of publication of application: **02.01.2019**
(21) Application number: 17382417.8
(22) Date of filing: 29.06.2017
(51) Int. Cl.: C12Q 1/68

(54) **IDENTIFICATION AND ELIMINATION OF DAMAGED AND/OR SENESCENT CELLS**

(71) Applicant: Fundación Centro Nacional de Investigaciones Oncológicas Carlos III, 28029 Madrid (ES)
(72) Inventor: SERRANO MARUGÁN, Manuel, 28760 Tres Cantos, Madrid (ES); LLANOS GIRÓN, Susana, 28049 Madrid (ES); CHAIB, Andy Selim, 53520 Wershofen (DE)
(74) Representative: Elzaburu S.L.P.

(57) **Abstract**

Identification and elimination of damaged and/or senescent cells.

This invention relates to methods of detecting cells over-expressing of Programmed Death Ligand 2 (PD-L2), in particular, damaged and/or senescent cells, and the identification thereof. The invention also extends to the use of antagonists of Programmed Death Ligand 2 (PD-L2) and Programmed Cell Death Protein 1 (PD-1) interaction, or inhibitors of PD-1 or PD-L2 expression to eliminate damaged and/or senescent cells, which are involved in numerous pathologies and aging, and are also produced as a result of exposure to toxic substances.

SEQUENCE LISTING

<110>  Fundación Centro Nacional de Investigaciones Oncológicas
       Carlos III

<120>  Identification and elimination of damaged and/or senescent cells

<130>  EP-1591

<160>  26

<170>  PatentIn version 3.5

<210>  1
<211>  2115
<212>  DNA
<213>  Homo sapiens

<220>
<221>  misc_feature
<222>  (1)..(2115)
<223>  DNA corresponding to mRNA of human PD-1 (NCBI accession number
       NM_005018)

<220>
<221>  CDS
<222>  (69)..(935)

<400>  1
agtttccctt ccgctcacct ccgcctgagc agtggagaag gcggcactct ggtggggctg      60

ctccaggc atg cag atc cca cag gcg ccc tgg cca gtc gtc tgg gcg gtg      110
         Met Gln Ile Pro Gln Ala Pro Trp Pro Val Val Trp Ala Val
         1               5                   10

cta caa ctg ggc tgg cgg cca gga tgg ttc tta gac tcc cca gac agg      158
Leu Gln Leu Gly Trp Arg Pro Gly Trp Phe Leu Asp Ser Pro Asp Arg
15                  20                  25                  30

ccc tgg aac ccc ccc acc ttc tcc cca gcc ctg ctc gtg gtg acc gaa      206
Pro Trp Asn Pro Pro Thr Phe Ser Pro Ala Leu Leu Val Val Thr Glu
                35                  40                  45

ggg gac aac gcc acc ttc acc tgc agc ttc tcc aac aca tcg gag agc      254
Gly Asp Asn Ala Thr Phe Thr Cys Ser Phe Ser Asn Thr Ser Glu Ser
            50                  55                  60

ttc gtg cta aac tgg tac cgc atg agc ccc agc aac cag acg gac aag      302
Phe Val Leu Asn Trp Tyr Arg Met Ser Pro Ser Asn Gln Thr Asp Lys
        65                  70                  75

ctg gcc gcc ttc ccc gag gac cgc agc cag ccc ggc cag gac tgc cgc      350
Leu Ala Ala Phe Pro Glu Asp Arg Ser Gln Pro Gly Gln Asp Cys Arg
    80                  85                  90

ttc cgt gtc aca caa ctg ccc aac ggg cgt gac ttc cac atg agc gtg      398
Phe Arg Val Thr Gln Leu Pro Asn Gly Arg Asp Phe His Met Ser Val
95                  100                 105                 110

gtc agg gcc cgg cgc aat gac agc ggc acc tac ctc tgt ggg gcc atc      446
Val Arg Ala Arg Arg Asn Asp Ser Gly Thr Tyr Leu Cys Gly Ala Ile
                115                 120                 125

tcc ctg gcc ccc aag gcg cag atc aaa gag agc ctg cgg gca gag ctc     494
Ser Leu Ala Pro Lys Ala Gln Ile Lys Glu Ser Leu Arg Ala Glu Leu
            130                 135                 140

agg gtg aca gag aga agg gca gaa gtg ccc aca gcc cac ccc agc ccc     542
Arg Val Thr Glu Arg Arg Ala Glu Val Pro Thr Ala His Pro Ser Pro
        145                 150                 155

tca ccc agg cca gcc ggc cag ttc caa acc ctg gtg gtt ggt gtc gtg     590
Ser Pro Arg Pro Ala Gly Gln Phe Gln Thr Leu Val Val Gly Val Val
    160                 165                 170

ggc ggc ctg ctg ggc agc ctg gtg ctg cta gtc tgg gtc ctg gcc gtc     638
Gly Gly Leu Leu Gly Ser Leu Val Leu Leu Val Trp Val Leu Ala Val
175                 180                 185                 190

atc tgc tcc cgg gcc gca cga ggg aca ata gga gcc agg cgc acc ggc     686
Ile Cys Ser Arg Ala Ala Arg Gly Thr Ile Gly Ala Arg Arg Thr Gly
                195                 200                 205

cag ccc ctg aag gag gac ccc tca gcc gtg cct gtg ttc tct gtg gac     734
Gln Pro Leu Lys Glu Asp Pro Ser Ala Val Pro Val Phe Ser Val Asp
            210                 215                 220

tat ggg gag ctg gat ttc cag tgg cga gag aag acc ccg gag ccc ccc     782
Tyr Gly Glu Leu Asp Phe Gln Trp Arg Glu Lys Thr Pro Glu Pro Pro
        225                 230                 235

gtg ccc tgt gtc cct gag cag acg gag tat gcc acc att gtc ttt cct     830
Val Pro Cys Val Pro Glu Gln Thr Glu Tyr Ala Thr Ile Val Phe Pro
    240                 245                 250

agc gga atg ggc acc tca tcc ccc gcc cgc agg ggc tca gct gac ggc     878
Ser Gly Met Gly Thr Ser Ser Pro Ala Arg Arg Gly Ser Ala Asp Gly
255                 260                 265                 270

cct cgg agt gcc cag cca ctg agg cct gag gat gga cac tgc tct tgg     926
Pro Arg Ser Ala Gln Pro Leu Arg Pro Glu Asp Gly His Cys Ser Trp
                275                 280                 285

ccc ctc tga ccggcttcct tggccaccag tgttctgcag accctccacc     975
Pro Leu

atgagcccgg gtcagcgcat ttcctcagga gaagcaggca gggtgcaggc cattgcaggc     1035

cgtccagggg ctgagctgcc tgggggcgac cggggctcca gcctgcacct gcaccaggca     1095

cagccccacc acaggactca tgtctcaatg cccacagtga gcccaggcag caggtgtcac     1155

cgtcccctac agggagggcc agatgcagtc actgcttcag gtcctgccag cacagagctg     1215

cctgcgtcca gctccctgaa tctctgctgc tgctgctgct gctgctgctg ctgcctgcgg     1275

cccggggctg aaggcgccgt ggccctgcct gacgccccgg agcctcctgc ctgaacttgg     1335

gggctggttg gagatggcct tggagcagcc aaggtgcccc tggcagtggc atcccgaaac     1395

gccctggacg cagggcccaa gactgggcac aggagtggga ggtacatggg gctggggact     1455

ccccaggagt tatctgctcc ctgcaggcct agagaagttt cagggaaggt cagaagagct     1515

cctggctgtg gtgggcaggg caggaaaccc ctccaccttt acacatgccc aggcagcacc 1575

tcaggccctt tgtggggcag ggaagctgag gcagtaagcg ggcaggcaga gctggaggcc 1635

tttcaggccc agccagcact ctggcctcct gccgccgcat tccaccccag cccctcacac 1695

cactcgggag agggacatcc tacggtccca aggtcaggag ggcagggctg gggttgactc 1755

aggcccctcc cagctgtggc cacctgggtg ttgggagggc agaagtgcag gcacctaggg 1815

ccccccatgt gcccaccctg ggagctctcc ttggaaccca ttcctgaaat tatttaaagg 1875

ggttggccgg gctcccacca gggcctgggt gggaaggtac aggcgttccc ccggggccta 1935

gtacccccgc cgtggcctat ccactcctca catccacaca ctgcaccccc actcctgggg 1995

cagggccacc agcatccagg cggccagcag gcacctgagt ggctgggaca agggatcccc 2055

cttccctgtg gttctattat attataatta taattaaata tgagagcatg ctaaggaaaa 2115

<210> 2
<211> 288
<212> PRT
<213> Homo sapiens

<400> 2

Met Gln Ile Pro Gln Ala Pro Trp Pro Val Val Trp Ala Val Leu Gln
1               5                   10                  15

Leu Gly Trp Arg Pro Gly Trp Phe Leu Asp Ser Pro Asp Arg Pro Trp
            20                  25                  30

Asn Pro Pro Thr Phe Ser Pro Ala Leu Leu Val Val Thr Glu Gly Asp
        35                  40                  45

Asn Ala Thr Phe Thr Cys Ser Phe Ser Asn Thr Ser Glu Ser Phe Val
    50                  55                  60

Leu Asn Trp Tyr Arg Met Ser Pro Ser Asn Gln Thr Asp Lys Leu Ala
65                  70                  75                  80

Ala Phe Pro Glu Asp Arg Ser Gln Pro Gly Gln Asp Cys Arg Phe Arg
                85                  90                  95

Val Thr Gln Leu Pro Asn Gly Arg Asp Phe His Met Ser Val Val Arg
            100                 105                 110

Ala Arg Arg Asn Asp Ser Gly Thr Tyr Leu Cys Gly Ala Ile Ser Leu
        115                 120                 125

Ala Pro Lys Ala Gln Ile Lys Glu Ser Leu Arg Ala Glu Leu Arg Val
    130                 135                 140

Thr Glu Arg Arg Ala Glu Val Pro Thr Ala His Pro Ser Pro Ser Pro
145                 150                 155                 160

Arg Pro Ala Gly Gln Phe Gln Thr Leu Val Val Gly Val Val Gly Gly
                165                 170                 175

Leu Leu Gly Ser Leu Val Leu Leu Val Trp Val Leu Ala Val Ile Cys
            180                 185                 190

Ser Arg Ala Ala Arg Gly Thr Ile Gly Ala Arg Arg Thr Gly Gln Pro
        195                 200                 205

Leu Lys Glu Asp Pro Ser Ala Val Pro Val Phe Ser Val Asp Tyr Gly
    210                 215                 220

Glu Leu Asp Phe Gln Trp Arg Glu Lys Thr Pro Glu Pro Pro Val Pro
225                 230                 235                 240

Cys Val Pro Glu Gln Thr Glu Tyr Ala Thr Ile Val Phe Pro Ser Gly
                245                 250                 255

Met Gly Thr Ser Ser Pro Ala Arg Arg Gly Ser Ala Asp Gly Pro Arg
            260                 265                 270

Ser Ala Gln Pro Leu Arg Pro Glu Asp Gly His Cys Ser Trp Pro Leu
        275                 280                 285

<210>  3
<211>  2418
<212>  DNA
<213>  Homo sapiens

<220>
<221>  misc_feature
<222>  (1)..(2418)
<223>  DNA coding mRNA of PD-L2 (GenBank accesion no. NM_025239, version
       NM_025239.3)

<220>
<221>  CDS
<222>  (274)..(1095)
<223>  CDS of PD-L2 human protein

<400>  3
gcaaacctta agctgaatga acaacttttc ttctcttgaa tatatcttaa cgccaaattt      60

tgagtgcttt tttgttaccc atcctcatat gtcccagcta gaaagaatcc tgggttggag     120

ctactgcatg ttgattgttt tgtttttcct tttggctgtt cattttggtg gctactataa     180

ggaaatctaa cacaaacagc aactgttttt tgttgtttac ttttgcatct ttacttgtgg     240

agctgtggca agtcctcata tcaaatacag aac atg atc ttc ctc ctg cta atg 294
Met Ile Phe Leu Leu Leu Met
1               5

ttg agc ctg gaa ttg cag ctt cac cag ata gca gct tta ttc aca gtg 342
Leu Ser Leu Glu Leu Gln Leu His Gln Ile Ala Ala Leu Phe Thr Val
        10                  15                  20

aca gtc cct aag gaa ctg tac ata ata gag cat ggc agc aat gtg acc 390
Thr Val Pro Lys Glu Leu Tyr Ile Ile Glu His Gly Ser Asn Val Thr
    25                  30                  35

ctg gaa tgc aac ttt gac act gga agt cat gtg aac ctt gga gca ata 438
Leu Glu Cys Asn Phe Asp Thr Gly Ser His Val Asn Leu Gly Ala Ile
40                  45                  50                  55

aca gcc agt ttg caa aag gtg gaa aat gat aca tcc cca cac cgt gaa 486
Thr Ala Ser Leu Gln Lys Val Glu Asn Asp Thr Ser Pro His Arg Glu
                60                  65                  70

aga gcc act ttg ctg gag gag cag ctg ccc cta ggg aag gcc tcg ttc 534
Arg Ala Thr Leu Leu Glu Glu Gln Leu Pro Leu Gly Lys Ala Ser Phe
            75                  80                  85

cac ata cct caa gtc caa gtg agg gac gaa gga cag tac caa tgc ata 582
His Ile Pro Gln Val Gln Val Arg Asp Glu Gly Gln Tyr Gln Cys Ile
        90                  95                  100

atc atc tat ggg gtc gcc tgg gac tac aag tac ctg act ctg aaa gtc 630
Ile Ile Tyr Gly Val Ala Trp Asp Tyr Lys Tyr Leu Thr Leu Lys Val
    105                 110                 115

aaa gct tcc tac agg aaa ata aac act cac atc cta aag gtt cca gaa 678
Lys Ala Ser Tyr Arg Lys Ile Asn Thr His Ile Leu Lys Val Pro Glu
120                 125                 130                 135

aca gat gag gta gag ctc acc tgc cag gct aca ggt tat cct ctg gca 726
Thr Asp Glu Val Glu Leu Thr Cys Gln Ala Thr Gly Tyr Pro Leu Ala
                140                 145                 150

gaa gta tcc tgg cca aac gtc agc gtt cct gcc aac acc agc cac tcc 774
Glu Val Ser Trp Pro Asn Val Ser Val Pro Ala Asn Thr Ser His Ser
            155                 160                 165

agg acc cct gaa ggc ctc tac cag gtc acc agt gtt ctg cgc cta aag 822
Arg Thr Pro Glu Gly Leu Tyr Gln Val Thr Ser Val Leu Arg Leu Lys
        170                 175                 180

cca ccc cct ggc aga aac ttc agc tgt gtg ttc tgg aat act cac gtg 870
Pro Pro Pro Gly Arg Asn Phe Ser Cys Val Phe Trp Asn Thr His Val
    185                 190                 195

agg gaa ctt act ttg gcc agc att gac ctt caa agt cag atg gaa ccc 918
Arg Glu Leu Thr Leu Ala Ser Ile Asp Leu Gln Ser Gln Met Glu Pro
200                 205                 210                 215

agg acc cat cca act tgg ctg ctt cac att ttc atc ccc ttc tgc atc 966
Arg Thr His Pro Thr Trp Leu Leu His Ile Phe Ile Pro Phe Cys Ile
                220                 225                 230

att gct ttc att ttc ata gcc aca gtg ata gcc cta aga aaa caa ctc 1014
Ile Ala Phe Ile Phe Ile Ala Thr Val Ile Ala Leu Arg Lys Gln Leu
            235                 240                 245

tgt caa aag ctg tat tct tca aaa gac aca aca aaa aga cct gtc acc     1062
Cys Gln Lys Leu Tyr Ser Ser Lys Asp Thr Thr Lys Arg Pro Val Thr
        250                 255                 260

aca aca aag agg gaa gtg aac agt gct atc tga acctgtggtc ttgggagcca     1115
Thr Thr Lys Arg Glu Val Asn Ser Ala Ile
    265                 270

gggtgacctg atatgacatc taaagaagct tctggactct gaacaagaat tcggtggcct     1175

gcagagcttg ccatttgcac ttttcaaatg cctttggatg acccagcact ttaatctgaa     1235

acctgcaaca agactagcca acacctggcc atgaaacttg ccccttcact gatctggact     1295

cacctctgga gcctatggct ttaagcaagc actactgcac tttacagaat taccccactg     1355

gatcctggac ccacagaatt ccttcaggat ccttcttgct gccagactga aagcaaaagg     1415

aattatttcc cctcaagttt tctaagtgat ttccaaaagc agaggtgtgt ggaaatttcc     1475

agtaacagaa acagatgggt tgccaataga gttatttttt atctatagct tcctctgggt     1535

actagaagag gctattgaga ctatgagctc acagacaggg cttcgcacaa actcaaatca     1595

taattgacat gttttatgga ttactggaat cttgatagca taatgaagtt gttctaatta     1655

acagagagca tttaaatata cactaagtgc acaaattgtg gagtaaagtc atcaagctct     1715

gtttttgagg tctaagtcac aaagcatttg ttttaacctg taatggcacc atgtttaatg     1775

gtggtttttt ttttgaacta catctttcct ttaaaaatta ttggtttctt tttatttgtt     1835

tttaccttag aaatcaatta tatacagtca aaaatatttg atatgctcat acgttgtatc     1895

tgcagcaatt tcagataagt agctaaaatg gccaaagccc caaactaagc ctccttttct     1955

ggccctcaat atgactttaa atttgacttt tcagtgcctc agtttgcaca tctgtaatac     2015

agcaatgcta agtagtcaag gcctttgata attggcacta tggaaatcct gcaagatccc     2075

actacatatg tgtggagcag aagggtaact cggctacagt aacagcttaa ttttgttaaa     2135

tttgttcttt atactggagc catgaagctc agagcattag ctgacccttg aactattcaa     2195

atgggcacat tagctagtat aacagactta cataggtggg cctaaagcaa gctccttaac     2255

tgagcaaaat ttggggctta tgagaatgaa agggtgtgaa attgactaac agacaaatca     2315

tacatctcag tttctcaatt ctcatgtaaa tcagagaatg cctttaaaga ataaaactca     2375

attgttattc ttcaacgttc tttatatatt ctacttttgg gta                       2418

<210>  4
<211>  273
<212>  PRT
<213>  Homo sapiens

<400>  4

Met Ile Phe Leu Leu Leu Met Leu Ser Leu Glu Leu Gln Leu His Gln
1               5                   10                  15

Ile Ala Ala Leu Phe Thr Val Thr Val Pro Lys Glu Leu Tyr Ile Ile
            20                  25                  30

Glu His Gly Ser Asn Val Thr Leu Glu Cys Asn Phe Asp Thr Gly Ser
        35                  40                  45

His Val Asn Leu Gly Ala Ile Thr Ala Ser Leu Gln Lys Val Glu Asn
    50                  55                  60

Asp Thr Ser Pro His Arg Glu Arg Ala Thr Leu Leu Glu Glu Gln Leu
65                  70                  75                  80

Pro Leu Gly Lys Ala Ser Phe His Ile Pro Gln Val Gln Val Arg Asp
                85                  90                  95

Glu Gly Gln Tyr Gln Cys Ile Ile Ile Tyr Gly Val Ala Trp Asp Tyr
            100                 105                 110

Lys Tyr Leu Thr Leu Lys Val Lys Ala Ser Tyr Arg Lys Ile Asn Thr
        115                 120                 125

His Ile Leu Lys Val Pro Glu Thr Asp Glu Val Glu Leu Thr Cys Gln
    130                 135                 140

Ala Thr Gly Tyr Pro Leu Ala Glu Val Ser Trp Pro Asn Val Ser Val
145                 150                 155                 160

Pro Ala Asn Thr Ser His Ser Arg Thr Pro Glu Gly Leu Tyr Gln Val
                165                 170                 175

Thr Ser Val Leu Arg Leu Lys Pro Pro Pro Gly Arg Asn Phe Ser Cys
            180                 185                 190

Val Phe Trp Asn Thr His Val Arg Glu Leu Thr Leu Ala Ser Ile Asp
        195                 200                 205

Leu Gln Ser Gln Met Glu Pro Arg Thr His Pro Thr Trp Leu Leu His
    210                 215                 220

Ile Phe Ile Pro Phe Cys Ile Ile Ala Phe Ile Phe Ile Ala Thr Val
225                 230                 235                 240

Ile Ala Leu Arg Lys Gln Leu Cys Gln Lys Leu Tyr Ser Ser Lys Asp
                245                 250                 255

Thr Thr Lys Arg Pro Val Thr Thr Thr Lys Arg Glu Val Asn Ser Ala

260                 265                 270

Ile

<210>  5
<211>  21
<212>  DNA
<213>  Artificial Sequence

<220>
<223>  human beta-actin forward primer

<400>  5
caaggccaac cgcgagaaga t                                                21

<210>  6
<211>  23
<212>  DNA
<213>  Artificial Sequence

<220>
<223>  human beta-actin reverse primer

<400>  6
ccagaggcgt acagggatag cac                                              23

<210>  7
<211>  21
<212>  DNA
<213>  Artificial Sequence

<220>
<223>  human PD-L1 forward primer

<400>  7
tggcatttgc tgaacgcatt t                                                21

<210>  8
<211>  22
<212>  DNA
<213>  Artificial Sequence

<220>
<223>  human PD-L1 reverse primer

<400>  8
tgcagccagg tctaattgtt tt                                               22

<210>  9
<211>  21
<212>  DNA
<213>  Artificial Sequence

<220>
<223>  human PD-L2 forward primer

<400>  9
attgcagctt caccagatag c                                                21

<210>  10
<211>  21
<212>  DNA
<213>  Artificial Sequence

<220>
<223>  human PD-L2 reverse primer

<400>  10
aaagttgcat tccagggtca c                                                21

<210>  11
<211>  18
<212>  DNA
<213>  Artificial Sequence

<220>
<223>  human TBP forward primer

<400>  11
atcagtgccg tggttcgt                                                    18

<210>  12
<211>  20
<212>  DNA
<213>  Artificial Sequence

<220>
<223>  human TBP reverse primer

<400>  12
ttcggagagt tctgggattg                                                  20

<210>  13
<211>  21
<212>  DNA
<213>  Artificial Sequence

<220>
<223>  mouse actin forward primer

<400>  13
ggcaccacac cttctacaat g                                                21

<210>  14
<211>  20
<212>  DNA
<213>  Artificial Sequence

<220>
<223>  mouse actin reverse primer

<400>  14
gtggtggtga agctgtagcc                                                  20

<210>  15
<211>  20
<212>  DNA
<213>  Artificial Sequence

<220>
<223>  mouse GAPDH forward primer

<400>  15
ttcaccacca tggagaaggc                                                   20


<210>  16
<211>  18
<212>  DNA
<213>  Artificial Sequence

<220>
<223>  mouse GAPDH reverse primer

<400>  16
ccctttggc tccaccct                                                      18


<210>  17
<211>  19
<212>  DNA
<213>  Artificial Sequence

<220>
<223>  mouse PD-L1 forward primer

<400>  17
agtcaatgcc ccataccgc                                                    19


<210>  18
<211>  20
<212>  DNA
<213>  Artificial Sequence

<220>
<223>  mouse PD-L1 reverse primer

<400>  18
ttctggataa ccctcggcct                                                   20


<210>  19
<211>  20
<212>  DNA
<213>  Artificial Sequence

<220>
<223>  mouse PD-L2 forward primer

<400>  19
caagcctcag cctagcagaa                                                   20


<210>  20
<211>  20

<212>  DNA
<213>  Artificial Sequence

<220>
<223>  mouse PD-L2 reverse primer

<400>  20
tccatccgac tcagagggtc                                                    20


<210>  21
<211>  20
<212>  DNA
<213>  Artificial Sequence

<220>
<223>  mouse IL-6 forward primer

<400>  21
gttctctggg aaatcgtgga                                                    20


<210>  22
<211>  22
<212>  DNA
<213>  Artificial Sequence

<220>
<223>  mouse IL-6 reverse primer

<400>  22
ggtactccag aagaccagag ga                                                 22


<210>  23
<211>  19
<212>  DNA
<213>  Artificial Sequence

<220>
<223>  mouse p21 forward primer

<400>  23
gtgggtctga ctccagccc                                                     19


<210>  24
<211>  20
<212>  DNA
<213>  Artificial Sequence

<220>
<223>  mouse p21 reverse primer

<400>  24
ccttctcgtg agacgcttac                                                    20


<210>  25
<211>  3415
<212>  DNA
<213>  Mus musculus

<220>
<221>  misc_feature
<222>  (1)..(3415)
<223>  DNA coding mRNA of PD-L2 (GenBank accesion no. NM_021396, version
       NM_021396.2)

<220>
<221>  CDS
<222>  (237)..(980)
<223>  Coding sequence of mouse PD-L2 protein

<400>  25
gaccacatca tttttgttcc ctttgttgga tatatcctaa tgtcaaatgt ggcatatctt      60

tgttgtctcc ttctgtctcc caactagaga gaacacactt acggctcctg tcccgggcag     120

gtttggttgt cggtgtgatt ggcttccagg gaacctgata caaggagcaa ctgtgtgctg     180

ccttttctgt gtctttgctt gaggagctgt gctgggtgct gatattgaca cagacc atg     239
                                                              Met
                                                              1

ctg ctc ctg ctg ccg ata ctg aac ctg agc tta caa ctt cat cct gta       287
Leu Leu Leu Leu Pro Ile Leu Asn Leu Ser Leu Gln Leu His Pro Val
            5                   10                  15

gca gct tta ttc acc gtg aca gcc cct aaa gaa gtg tac acc gta gac       335
Ala Ala Leu Phe Thr Val Thr Ala Pro Lys Glu Val Tyr Thr Val Asp
        20                  25                  30

gtc ggc agc agt gtg agc ctg gag tgc gat ttt gac cgc aga gaa tgc       383
Val Gly Ser Ser Val Ser Leu Glu Cys Asp Phe Asp Arg Arg Glu Cys
    35                  40                  45

act gaa ctg gaa ggg ata aga gcc agt ttg cag aag gta gaa aat gat       431
Thr Glu Leu Glu Gly Ile Arg Ala Ser Leu Gln Lys Val Glu Asn Asp
50                  55                  60                  65

acg tct ctg caa agt gaa aga gcc acc ctg ctg gag gag cag ctg ccc       479
Thr Ser Leu Gln Ser Glu Arg Ala Thr Leu Leu Glu Glu Gln Leu Pro
                70                  75                  80

ctg gga aag gct ttg ttc cac atc cct agt gtc caa gtg aga gat tcc       527
Leu Gly Lys Ala Leu Phe His Ile Pro Ser Val Gln Val Arg Asp Ser
            85                  90                  95

ggg cag tac cgt tgc ctg gtc atc tgc ggg gcc gcc tgg gac tac aag       575
Gly Gln Tyr Arg Cys Leu Val Ile Cys Gly Ala Ala Trp Asp Tyr Lys
        100                 105                 110

tac ctg acg gtg aaa gtc aaa gct tct tac atg agg ata gac act agg       623
Tyr Leu Thr Val Lys Val Lys Ala Ser Tyr Met Arg Ile Asp Thr Arg
    115                 120                 125

atc ctg gag gtt cca ggt aca ggg gag gtg cag ctt acc tgc cag gct       671
Ile Leu Glu Val Pro Gly Thr Gly Glu Val Gln Leu Thr Cys Gln Ala
130                 135                 140                 145

aga ggt tat ccc cta gca gaa gtg tcc tgg caa aat gtc agt gtt cct       719
Arg Gly Tyr Pro Leu Ala Glu Val Ser Trp Gln Asn Val Ser Val Pro
                150                 155                 160

gcc aac acc agc cac atc agg acc ccc gaa ggc ctc tac cag gtc acc 767
Ala Asn Thr Ser His Ile Arg Thr Pro Glu Gly Leu Tyr Gln Val Thr
            165                 170                 175

agt gtt ctg cgc ctc aag cct cag cct agc aga aac ttc agc tgc atg 815
Ser Val Leu Arg Leu Lys Pro Gln Pro Ser Arg Asn Phe Ser Cys Met
        180                 185                 190

ttc tgg aat gct cac atg aag gag ctg act tca gcc atc att gac cct 863
Phe Trp Asn Ala His Met Lys Glu Leu Thr Ser Ala Ile Ile Asp Pro
    195                 200                 205

ctg agt cgg atg gaa ccc aaa gtc ccc aga acg tgg cca ctt cat gtt 911
Leu Ser Arg Met Glu Pro Lys Val Pro Arg Thr Trp Pro Leu His Val
210                 215                 220                 225

ttc atc ccg gcc tgc acc atc gct ttg atc ttc ctg gcc ata gtg ata 959
Phe Ile Pro Ala Cys Thr Ile Ala Leu Ile Phe Leu Ala Ile Val Ile
                230                 235                 240

atc cag aga aag agg atc tag gggaagctgt attacggaag aagatctgga 1010
Ile Gln Arg Lys Arg Ile
            245

cctgcggtct tgggagttgg aaggatctga tgggaaaccc tcaagagact tctggactca 1070

aagtgagaat cttgcaggac ctgccatttg cacttttgaa ccctttggac ggtgacccag 1130

ggctccgaag aggagcttgt aagactgaca atcttccctc tgtctcaaga ctctctgaac 1190

agcaagaccc caatggcact ttagacttac cctgggatcc ctggacccca gtgagggcct 1250

aaggctccta atgactttca gggtgagaac aaaaggaatt gctctccgcc ccacccccac 1310

ctcctgcttt ccgcagggag acatggaaat tcccagttac taaaatagat tgtcaataga 1370

gttatttata gccctcattt cctccgggga cttggaagct tcagacaggg tttttcataa 1430

acaaagtcat aactgatgtg ttttacagca tcctagaatc ctggcagcct ctgaagttct 1490

aattaactgg aagcatttaa gcaacacgtt aagtaccccc actgtggtat ttgtttctac 1550

ttttctgttt ttaaagtgtg agtcacaagg taattgttgt aacctgtgat atcactgttt 1610

cttgtgtctc ttctttcaac tacatctttt aaaacaaaac ggtgtggggt ttggttgttt 1670

tggtggtagt ggtagtgttt ctcagtggta tctccttaag aaaaaaaatc atcatgccag 1730

tgaattgttt cttcagccat ttcagatggg aagctggaat agcctgtccc ccaagctaag 1790

ccttcttccc tagctttctg cgtgatttta cattgagcat tcctgttgct ttgtttctat 1850

aactgtaatg tggtgatgtc attgttaggg cacttgaggg tgggcgttct ggaagtcctt 1910

tcaggttagt gtttgggggc agggttgctc agaatacata aaggtgctaa cttaaactgc 1970

agccatggag ctcagtgaat tcactaacct tcgggctgtc caaatgtgca cattagctac 2030

tgtgacccct gtaggttagg gagcctgaag ccagctcttt acctggtgtt tagactcagc 2090

agaatttgga gtcaatggga ccaaatggtt gtgaaattaa gatttgaagt gtgcatctta 2150

ttttatcacc atctgcccaa caaaacttca gaaaatgcct ttgaagcaca aaaatgtaat 2210

cgtttatgtg aaatctctga gttgcattta gatgcccatt gcagcaaggt ggctctctca 2270

cagattccac accttagcct aagataccag acagcaggac agagagaaaa gtccttcctg 2330

gtgtgcaaac ttccttacac tggacctcgc ctctcaggtg tgtgattggt aggccaaatc 2390

ccgatagcca atcggtgttg ggtgctttgt ctgctctact gggagtccag tggtacaatg 2450

gattctggca aaatgctgcc atcttggccc tcgctgggct gctttctagg atattcatag 2510

agaaagggcc gtccagatcc agtatcctaa aatcctgaga ggagaatata agttagtgtg 2570

tctcactata actatctcta tgatcggtca cattactatc taacagttac caaatactat 2630

atgcctaata ctggtaagca ttttatacac accattggat tgaatcctct caaaatcctc 2690

aaaaaggaag ttattaatac ctccataggc aaggagccca gaacccagag aggtcaggca 2750

gtctagttat agatgcctgc tttgtttaga agtgaacaag agcatcaaat tattaatgtg 2810

ccctggttat taatgcgccc tggttacctg ctggatggaa catcaaggtg gacttttggc 2870

agttgcatac acccagaggt attttggcta ttcacggatt aatttcacac gaagtgtttc 2930

agagacatgt gtaggggaag tccgggttca gggggcctaa gattcaaact ctagcttagc 2990

tacgtctgac ctccctaagc actaacttac tatcaaaaga atgagcagta aaagaatggt 3050

gtttactgcc tgcctttatc aggcagtgaa cgtgcagcgg gcaacgaatg cttgataagt 3110

gtgtgtcagt gtgaagtccc atgtaccagc cgctgtcccc actgcaaaag cagcagagcg 3170

ctcagacatc atcagctgat ttaccagcag cagatttctt cttctagtcc catccctgaa 3230

gaagcttcca gcctaggtac attgcatggg ctttgtgctc caggagttcc tacacagccc 3290

tcaacttcaa cacaggcaaa gtgcttactg atcctcatgt atcttacagg gtcccctcta 3350

cccacaatac ctcattgctg gaacttcaaa tcttcctgaa taaaagcttg cccgtggttt 3410

aatta 3415

<210> 26
<211> 247
<212> PRT
<213> Mus musculus

<400> 26

Met Leu Leu Leu Leu Pro Ile Leu Asn Leu Ser Leu Gln Leu His Pro
1               5                   10                  15

Val Ala Ala Leu Phe Thr Val Thr Ala Pro Lys Glu Val Tyr Thr Val
            20                  25                  30

Asp Val Gly Ser Ser Val Ser Leu Glu Cys Asp Phe Asp Arg Arg Glu
        35                  40                  45

Cys Thr Glu Leu Glu Gly Ile Arg Ala Ser Leu Gln Lys Val Glu Asn
    50                  55                  60

Asp Thr Ser Leu Gln Ser Glu Arg Ala Thr Leu Leu Glu Glu Gln Leu
65                  70                  75                  80

Pro Leu Gly Lys Ala Leu Phe His Ile Pro Ser Val Gln Val Arg Asp
                85                  90                  95

Ser Gly Gln Tyr Arg Cys Leu Val Ile Cys Gly Ala Ala Trp Asp Tyr
            100                 105                 110

Lys Tyr Leu Thr Val Lys Val Lys Ala Ser Tyr Met Arg Ile Asp Thr
        115                 120                 125

Arg Ile Leu Glu Val Pro Gly Thr Gly Glu Val Gln Leu Thr Cys Gln
    130                 135                 140

Ala Arg Gly Tyr Pro Leu Ala Glu Val Ser Trp Gln Asn Val Ser Val
145                 150                 155                 160

Pro Ala Asn Thr Ser His Ile Arg Thr Pro Glu Gly Leu Tyr Gln Val
                165                 170                 175

Thr Ser Val Leu Arg Leu Lys Pro Gln Pro Ser Arg Asn Phe Ser Cys
            180                 185                 190

Met Phe Trp Asn Ala His Met Lys Glu Leu Thr Ser Ala Ile Ile Asp
        195                 200                 205

Pro Leu Ser Arg Met Glu Pro Lys Val Pro Arg Thr Trp Pro Leu His
    210                 215                 220

Val Phe Ile Pro Ala Cys Thr Ile Ala Leu Ile Phe Leu Ala Ile Val
225                 230                 235                 240

Ile Ile Gln Arg Lys Arg Ile
                245

## Description

### FIELD OF THE INVENTION

This invention relates to damaged and/or senescent cells and the ways of detecting them and/or eliminating them. More specifically, the invention relates to a novel method of detecting and/or identifying damaged and/or senescent cells, as well as to method of eliminating such cells and, through it, treating or ameliorating pathologies where such cells are involved, even some aging effects, which are also produced as a result of exposure to toxic substances. The invention extends to methods of detecting cells expressing PD-L2, in particular, although not exclusively, damaged and/or senescent cells. This invention also relates to antagonists of Programmed Death Ligand 2 (PD-L2) and Programmed Cell Death Protein 1 (PD-1) interaction. The invention also extends to the use of these antagonists to eliminate damaged and/or senescent cells.

### BACKGROUND OF THE INVENTION

Damaged cells that cannot undergo repair activate two cellular programs: apoptosis or senescence. Cellular senescence is characterized by three properties: (1) an epigenetic reorganization that prevents further cell proliferation; (2) an intense secretory program or Senescent Associated Secretory Phenotype (SASP) that includes cytokines that recruit inflammatory cells; and (3) up-regulation of lysosomal-associated *β*-galactosidase activity (SA-*β* gal) and lysosomal-associated *α*-fructose (SA-*α*-fuc) activity. These properties are not necessarily linked. For example, in the absence of p53, damaged cells cannot undergo arrest but they can implement a secretory phenotype (Coppé *et al.,* 2008). On the other hand, SA-*β* gal, the most commonly used biomarker of cellular senescence, is often present under certain conditions of restricted cell proliferation, including DNA damage. Thus, the boundaries between cellular damage and cellular senescence are not distinctly defined.

Moreover, cellular damage and cellular senescence are closely associated concepts; damaged cells often become senescent cells. Conversely, cellular senescence (initiated by means that do not necessarily imply cellular damage) is characterized by aberrant mitochondrial function that results in high levels of reactive oxygen species (ROS) that self-inflict damage to the cell, thereby ensuring that senescent cells are indeed themselves damaged cells (Ziegler *et al.,* 2015).

Damaged and/or senescent cells are present in many human pathologies (Muñoz-Espin and Serrano, 2014), including, but not restricted to, pulmonary fibrosis, type 2 diabetes, sarcopenia, cataracts, radiation-induced oral mucositis, brain and heart aneurysms, glaucoma, Alzheimer's and Parkinsons' disease, cystic fibrosis, intestinal bowel disease, osteoarthritis and intervertebral disc degeneration (Muñoz-Espin and Serrano, 2014). The list of diseases associated with damaged and/or senescent cells keeps growing steadily and it would not be unreasonable to assume that senescence is implicated in further pathologies. Therefore, the development of reliable methods for the identification of damaged and/or senescent cells could be of prognostic value for these pathologies.

Senescence can also be induced in response to therapy-induced genotoxic stress. For example, when treated with radio/chemotherapeutic agents, tumor cells are severely damaged and subsequently a fraction of these cells die by apoptosis, while others undergo senescence (Ewald *et al.,* 2010; Sanoff *et al.,* 2014; Wood *et al.,* 2016). The presence of surviving damaged and/or senescent cells may promote metastasis (Ruhland *et al.,* 2016) and may also have a negative effect on the full regression of the tumor concerned (Sun *et al.,* 2012; Zacarias-Fluck *et al.,* 2015), thus facilitating tumor relapse. Moreover, cancer survivors that have been exposed to chemotherapy are known to develop, years after their treatment, a higher frequency of chronic health conditions compared to their non-exposed counterparts and have an increased mortality rate (Ness *et al.,* 2015). This is thought to be caused by the persistence of damaged and/or senescent cells that arise throughout the entire organism as a consequence of systemic exposure to the chemotherapeutic agents (Ewald *et al.,* 2010). Similarly, in a related aspect, exposure to environmental toxicants has also been found to result in the accumulation of damaged/senescent cells in mice (Sorrentino *et al.,* 2014).

Recently, selective inhibitors of CDK4 and CDK6 kinases have been developed. In some cancers, these inhibitors induce a senescent response in most tumor cells, and, as a consequence, they offer great promise as a treatment for cancer. One of these inhibitors, Palbociclib (PD-0332991), has been approved for clinical use in combination with Letrozole for the treatment of ER-positive and HER2-negative breast cancer. However, the occurrence of the aforementioned senescent cells, and the existence of side-effects associated with these cells, are a cause of concern for these senescent-inducing therapies and could reduce the efficacy of these drugs in treating cancer patients.

Senescent cells also accumulate in aged organisms and negatively influence lifespan. In fact, the selective elimination of senescent cells from transgenic progeroid mice and naturally-aged mice has been shown to delay tumorigenesis and lead to greater resistance to aging-associated disorders (Baker *et al.,* 2011, 2016; Childs *et al.,* 2016; Zhu *et al.,* 2015).

Consequently, interventions that reduce the burden of damaged and/or senescent cells could potentially ameliorate chronic diseases and age-related disabilities (Zhu *et al.,* 2015). For this reason, there is great interest in the identification of drugs, known as senolytics, that are able to selectively kill damaged and/or senescent cells.

Several drugs with senolytic activity have been identified and investigated with respect to their efficacy in extending lifespan and alleviating age-associated disorders in mouse models.

Examples of these senolytic drugs include Dasatinib, a tyrosine kinase inhibitor developed by Bristol-Myers Squibb for the treatment of chronic leukemia, and Quercetin, a flavonol that has phosphatidyl inositol 3 kinase (PI3K) inhibitory activity. Used in combination, these drugs reduced the number of damaged and/or senescent cells in chronologically aged mice, in progeroid mice, and in irradiation-exposed mice, thereby resulting in improved cardiac and vascular function, delayed aged-related symptoms and extended lifespan (Zhu *et al.,* 2015; Roos *et al.,* 2016). In addition, ABT-737, an inhibitor of the anti-apoptotic proteins BCL-W and BCL-XL, both of which are up-regulated by senescent cells, has been shown to selectively eliminate senescent cells and, as a result, rejuvenate hematopoietic stem cells in irradiated or naturally-aged mice (Yosef *et al.,* 2016). Another inhibitor of the Bcl-2 family, Navitoclax (ABT-263), has also been shown to efficiently eliminate senescent cells *in vitro* and *in vivo* (Chang *et al.,* 2016; Zhu *et al.,* 2016). Pharmacological elimination of senescent cells has also been shown to alleviate atherosclerosis symptoms (Childs *et al.,* 2016; Roos *et al.,* 2016) and disc degeneration (Chen *et al.,* 2016) in mice.

Although the above-mentioned therapies have been demonstrated to be effective in animal models with respect to damaged and/or senescent cells, they have never been tested to evaluate whether they have the same effectiveness in humans. Rather, they are currently only being evaluated in human clinical trials as anti-cancer therapies. The reason for this is that all of the above treatments also induce an apoptotic response in healthy cells, as well as tumor and/or damaged and/or senescent cells. This apoptotic response in healthy cells is commonly associated with most anti-cancer therapies and is responsible for the often debilitating side effects routinely associated with standard chemotherapy. Consequently, the overall benefit gained in using these therapies to treat chronic diseases, or as preventative interventions, should be carefully assessed.

There is, therefore, a need in the art for a method capable of eliminating damaged and/or senescent cells, but without the cytotoxic side-effects associated with the therapies referred to above. Preferably, such method should be applicable for the prevention and/or the treatment of pathologies or diseases which are associated to the presence of senescent cells.

Additionally, it must be commented that there is currently no universally accepted standard of evidence for identifying senescent cells and determining their functional significance *in vivo,* as it can be found mentioned in several references related to the matter, such as the review of the group of van Deursen (Childs *et al.,* 2015). The methods that are currently used for detecting the presence of senescent cells in biological samples (such as those based in the upregulation of p16 or p21 proteins, including those where reporter genes are linked to the promoters of the corresponding genes, as the one discussed in Box 1 of the review of the group of van Deursen above mentioned) are not specific to senescent cells or, as happens with the method based on the up-regulation of lysosomal-associated *β*-galactosidase activity (SA-*β* gal), they require that cells or tissues are fixed before applying the method, what hinders its applicability and makes almost impossible their use for *in vivo* detection methods. This last disadvantage is not so relevant for methods to be applied to human beings, but it becomes more important when the detection is envisaged as a research tool in animal models used for increasing the knowledge about the process of senescence, its involvement in certain pathologies and possible means or targets for their treatment. Thus, it would be also advisable to have available one method for the detection of senescent cells with increased specificity and, preferably, based on an activity or a change of expression of a gene that makes feasible its application *in vivo.* It should be also preferable that such method for the detection of damaged and/or senescent cells could be of prognostic value for one or more pathologies associated to the presence of said cells, especially for human beings, even when the method of detection is carried out on one or more biological sample taken from the subject.

The present inventors have found a way of overcoming said problems.

In order to properly explain the proposed solution, it is useful to remind that the activation of T cells requires at least two signals: the first signal, which confers specificity to the immune response, is transduced *via* the T cell receptor (TCR) following recognition of a foreign antigen presented by the major histocompatibility complex (MHC). The second signal, termed costimulation, induces T cells to proliferate and become functional. Costimulation of T cells is neither antigen-specific, nor MHC-restricted, and is, instead, caused by distinct cell surface molecules expressed by antigen presenting cells (APCs). In addition, inhibitory receptors have also been identified on T cells. One of these inhibitory receptors is Programmed Cell Death Protein 1 (PD-1). Interaction of PD-1 with any of its two ligands: Programmed Death-Ligands 1 and 2 (PD-L1 and PD-L2, also known as CD274 or B7-H1 and CD273 or B7-DC, respectively), results in reduced cytokine production and reduced T cell survival, thus reducing autoimmunity and promoting self-tolerance (Fagarasan and Honjo, 2000). Manipulation of the T cell costimulatory/inhibitory pathways, therefore, offers great potential to stimulate or suppress immune responses in humans and animals. The PD-1 receptor is also expressed by natural killer cells (NK) and its blockade by specific antibodies against this protein induces their expansion and enhances their cytotoxic activity (Guo *et al.,* 2016).

As well as T cells and NK cells, many tumor cells express PD-L1, and the role of PD-1 in cancer immune evasion is well established. Antibodies and peptides against PD-1 and PD-L1 have been developed for cancer therapy (Ohaegbulam *et al.,* 2015; Pardoll, 2012; Sharma and Allison, 2015) and have been proven to be very effective against certain types of cancers. Examples of therapeutic anti-PD-1 antibodies are Nivolumab (Bristol-Myers Squibb), Pembrolizumab (Merck) and Pidilizumab (Medivation), which are clinically approved for the treatment of metastatic melanoma, non-small cell lung cancer (NSCLC) and diffuse large cell lymphomas (DLBCL), respectively.

The possible role of PD-L2 in the induction of immune tolerance in the tumour microenvironment has received less attention, probably due to its low constitutive basal expression and its restricted expression, which initially was thought to be restricted to antigen-presenting cells such as macrophages and dendritic cells (DCs), although some groups have subsequently reported that PD-L1 expression can be induced in a wider variety of other immune cells and nonimmune cells depending on microenvironmental stimuli among which Th2 cytokines seem to be particularly important. As Rozali *et al.* concluded in their review article (Rozali *et al.,* 2012), there are data indicating that targeting PD-L2 in cancer may be a viable treatment approach, although most PD-L2 blocking studies have been carried out in combination with the targeting of PD-L1 (Panekh *et al.,* 2009; He *et al.,* 2004). Even its involvement in other immune responses is also not clear, although it is possible to find some reports about the effect of inhibiting its expression with specific siRNAs (see, for instance, Hobo *et al.,* 2010).

However, the present inventors have found unexpected evidences related to PD-L2 that help to provide solutions to the problems set forth above.

### SUMMARY OF THE INVENTION

The present inventors are the first to identify that senescent tumor cells and non-apoptotic damaged cells highly increase their transcriptional levels of *PDCD1LG2* gene (the gene of PD-L2 ligand) and the levels of its encoded protein, PD-L2. They have observed that *in vivo* procedures that result in DNA damage and/or senescence also lead to the up-regulation of PD-L2 protein and its associated mRNA levels. They have also observed the upregulation of PD-L2 in aging. The inventors conclude that damaged and/or senescent cells induce an immunosuppressive environment and evade the detection of the immune system *via* the inhibition of PD-1 mediated signaling, through the up-regulation of PD-L2.

The present inventors propose to take advantage of such finding to detect and/or identify damaged and/or senescent cells and, even, to eliminate them avoiding cytotoxic side-effects associated with the current therapies above referred. It should be possible to achieve this desired advantage by breaching the immunotolerance generated by these damaged and/or senescent cells through the inhibition of the PD-L2/PD-1 signaling. In particular, a method based on the decrease or inhibition or the PD-L2/PD-1 signaling would result in the re-activation of immune cells and the clearance of damaged and/or senescent cells by the immune system, what should lead to an amelioration of pathologies where damaged and/or senescent cells are involved. The detection of damaged and/or senescent cells could also be useful in the prognostic/ diagnostic of the pathologies that are associated to the presence of said cells.

Until now, it could be considered that there were no specific methods available to detect damaged and/or senescent cell *in vitro* or *in vivo.* As a result of the inventors' observation that damaged and/or senescent cells upregulate their expression of PD-L2, it is now possible, for the first time, to identify the aforementioned cells *in vitro* or *in vivo* by analyzing PD-L2 mRNA levels or PD-L2 protein levels or, taking advantage of the low level of expression of PD-L2 in most cells in normal conditions, by using a molecule that binds to PD-L2 and detecting the binding of the molecule to PD-L2. This in turn facilitates the diagnosis of chronic diseases and conditions that result from the accumulation of such cells, which diseases and conditions might be, for example, those referred to above.

Consequently, the present inventors propose that a) the up-regulation of PD-L2 in damaged and/or senescent cells allows the detection and/or the identification of these cells; b) the detection of the presence of damaged and/or senescent cells that could be of prognostic value for the pathologies that are associated to the presence of these cells; c) the inhibition of PD-L2/PD-1 signaling may enhance immunity and contribute to the immunoclearance/elimination of damaged cells and senescent cells; and d) the clearance/elimination of damaged cells through the inhibition of the PD-L2/PD-1 signaling pathway should have a therapeutic effect on pathologies associated with the presence of damaged and/or senescent cells.

Thus, in a first aspect of the invention, there is provided a method for the detection of the presence of damaged and/or senescent cells in a sample, wherein the levels of a product (mRNA or protein) of the PD-L2 gene are determined and the presence of damaged and/or senescent cells is acknowledged when the levels are higher than a reference value.

In a second aspect, there is provided a method for the prognostic/diagnostic of a disease associated to the presence of damaged and/or senescent cells, wherein the presence of damaged and/or senescent cells is determined by the method of the first aspect of the invention.

In a third aspect, there is provided a method of eliminating damaged and/or senescent cells, the method comprising contacting the cells with an antagonist of the PD-L2/PD-1 interaction.

In a fourth aspect, the invention refers to an antagonist of the PD-L2/PD-1 interaction for use in eliminating damaged and/or senescent cells.

In a fifth aspect, strongly associated with the previous one, so that it can be considered a possible embodiment of it, the invention relates to an antagonist of the PD-L2/PD-1 interaction for use in treating, ameliorating or preventing a disease associated to the presence of damaged and/or senescent cells.

In a further aspect, there is provided a method of treating, ameliorating or preventing a disease characterised by the presence of damaged and/or senescent cells, the method comprising administering to a subject in need of such treatment, a therapeutically effective amount of antagonist/inhibitor of the PD-L2/PD-1 interaction.

In yet another aspect, there is provided a method of identifying damaged and/or senescent cells, the method comprising administering a molecule that binds to PD-L2 protein or PD-L2 mRNA, detecting whether the binding of the molecule to PD-L2 or PD-L2 mRNA and identifying as damaged or senescent cells those cells where the binding of the molecule to PD-L2 or PD-L2 mRNA can be detected or where the level of bound molecule is higher than a control value.

In another aspect of the invention, there is provided a composition which may advantageously be used in treating damaged and/or senescent cells, the composition, comprising an antagonist of the PD-L2/PD-1 interaction and a pharmaceutically acceptable vehicle.

The invention also provides in another aspect, a process for making the damaged and/or senescent cell-treatment composition according to the fourth aspect, the process comprising contacting a therapeutically effective amount of an antagonist of the PD-L2/PD-1 interaction and a pharmaceutically acceptable vehicle.

According to a another aspect of the invention, there is provided a monoclonal antibody that is an antagonist of the PD-12/PD-1 interaction.

Preferably, the antibody is an antibody or an antigen-binding fragment thereof that specifically binds to PD-L2 and/or PD-1 and interferes with the interaction between these two proteins. Even more preferably, the antibody is an antibody or an antigen-binding fragment thereof that specifically binds to PD-L2 and blocks its binding to its receptor PD-1.

Thus, in another aspect of the invention, there is provided a molecule that binds to PD-L2 or to PD-L2 mRNA for use in identifying damaged and/or senescent cells.

### BRIEF DESCRIPTION OF THE DRAWINGS

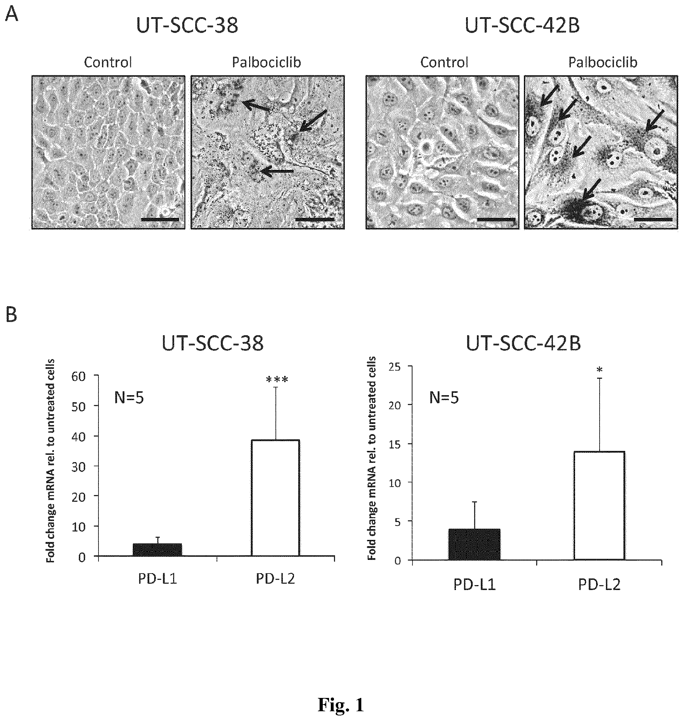

**Fig. 1** illustrates that PD-L2 mRNA is up-regulated in senescent head and neck squamous cell carcinoma (HNSCC) cells. (A) Representative images illustrating the SA-β galactosidase activity of the human HNSCC cell lines UT-SCC-38 and UT-SCC-42B treated with Palbociclib, a CDK4/CDK6 specific inhibitor, for 7 days. Palbociclib-treated showed a dramatically increased cellular size and accumulation of peri-nuclear granule that displayed a blue colour as a consequence of the increased SA-β galactosidase activity (arrows). Scale bar, 100 µm. (B) PD-L1 and PD-L2 mRNA levels were assessed by quantitative RT-PCR and normalised to the mRNA levels of the housekeeping gene *β-actin.* The data corresponds to the average +/- SD of five independent experiments and shows the fold change in PD-L1 mRNA levels and PD-L2 mRNA levels relative to untreated cells. Statistical t-test analysis was performed to calculate significance (**P≤*0.05; ****P*≤0.001);

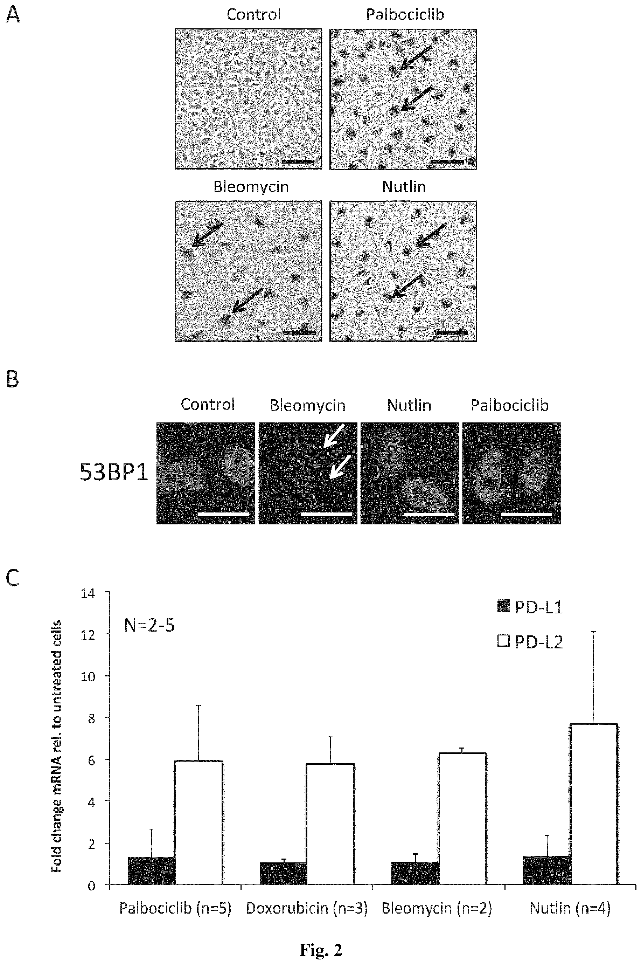

**Fig. 2** illustrates that PD-L2 mRNA is up-regulated in senescent melanoma cells. (A) Representative images illustrating the SA-β galactosidase activity of human melanoma SK-Mel-103 cells treated with either the CDK4/CDK6 inhibitor Palbociclib, the genotoxic drug Bleomycin or the Mdm2 inhibitor Nutlin for 7 days. Senescent cells showed a dramatically increased cellular size and accumulation of peri-nuclear granule that displayed a blue colour as a consequence of the increased SA□□ galactosidase activity (arrows). Scale bar, 100 µm. (B) Cells in (A) were immunostained with anti-53BP1 antibody and representative images were taken by confocal microscopy. Only bleomycin-treated cells exhibited nuclear 53-BP1 foci that reveal the presence of DNA double-strand breaks (arrows). Scale bar, 50 µm. (C) PD-L1 and PD-L2 mRNA levels were assessed by quantitative RT-PCR and normalised to the mRNA levels of the housekeeping gene *β-action.* The data corresponds to the average of two to five independent experiments +/- SD and shows the fold change in PD-L1 and PD-L2 mRNA levels in cells treated with Palbociclib, Doxorubicin, Bleomycin and Nutlin relative to untreated cells

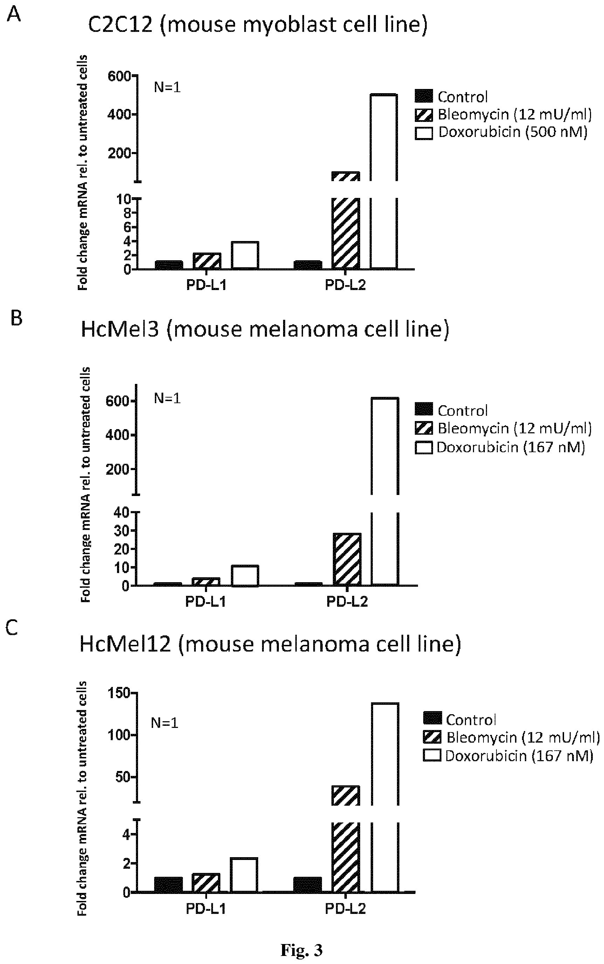

**Fig. 3** illustrates that DNA damage is a potent inducer of PD-L2 expression. The mouse myoblast cell line C2C12 (A) and the mouse melanoma cell lines HcMel3 (B) and HcMel12 (C) were treated with the genotoxic drugs Bleomycin and Doxorubicin at the indicated concentrations. 48 hours later PD-L1 and PD-L2 mRNA levels were analysed by quantitative RT-PCR. Data was normalised to the mRNA levels of the housekeeping gene *β-actin.* The graph shows the fold change in PD-L1 and PD-L2 mRNA levels relative to untreated cells. In each group of three, the left bar corresponds to control, the middle one to bleomycin and the right one to doxorubicin. This experiment has been done once.

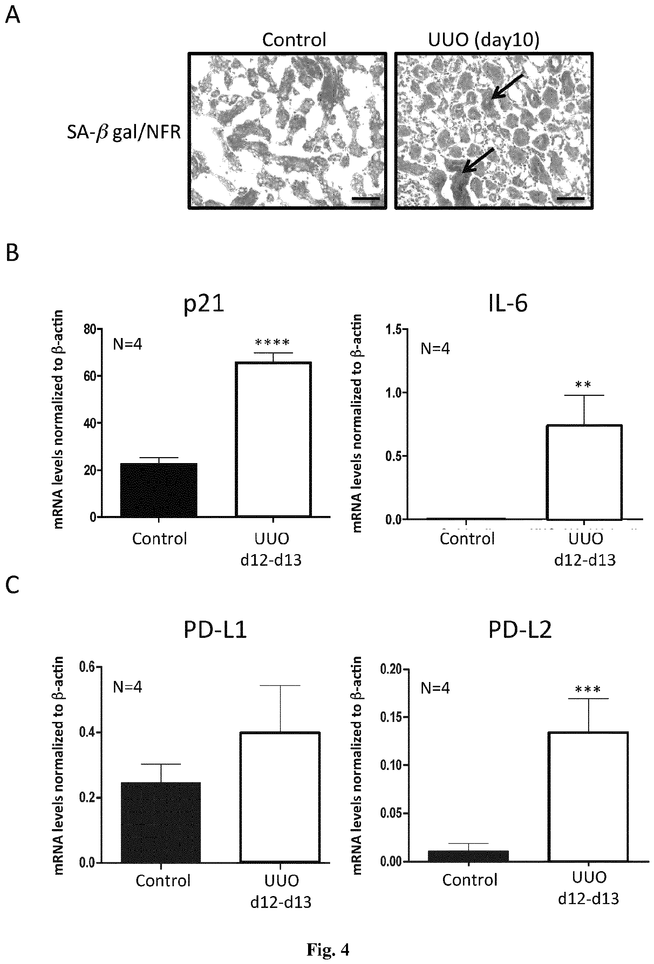

**Fig. 4** illustrates that PD-L2 expression is up-regulated in kidney fibrosis. Fibrosis was induced in C57BL6 mice kidneys by unilateral ureteral obstruction (UUO). (A) The presence of senescent cells was evaluated by SA-*β* galactosidase staining of optimal cutting temperature (OCT) compound impregnated tissue sections. In addition, cell nuclei were stained with Nuclear Red Fast (NRF). Senescent cells displayed a blue colour as a consequence of the increased SA-*β* galactosidase activity (arrows). Twelve to thirteen days post-treatment, the mRNA levels of senescent-associated genes p21 and IL-6 (B) and of the immune ligands PD-L1 and PD-L2 (C) were analysed by quantitative RT-PCR. Data was normalised to the mRNA levels of the housekeeping control gene *β-actin.* The data corresponds to the average +/- SD of four kidneys. Statistical t-test analysis was performed to calculate significance (**P≤0.01; ****P*≤0.001; *****P*≤0.0001);

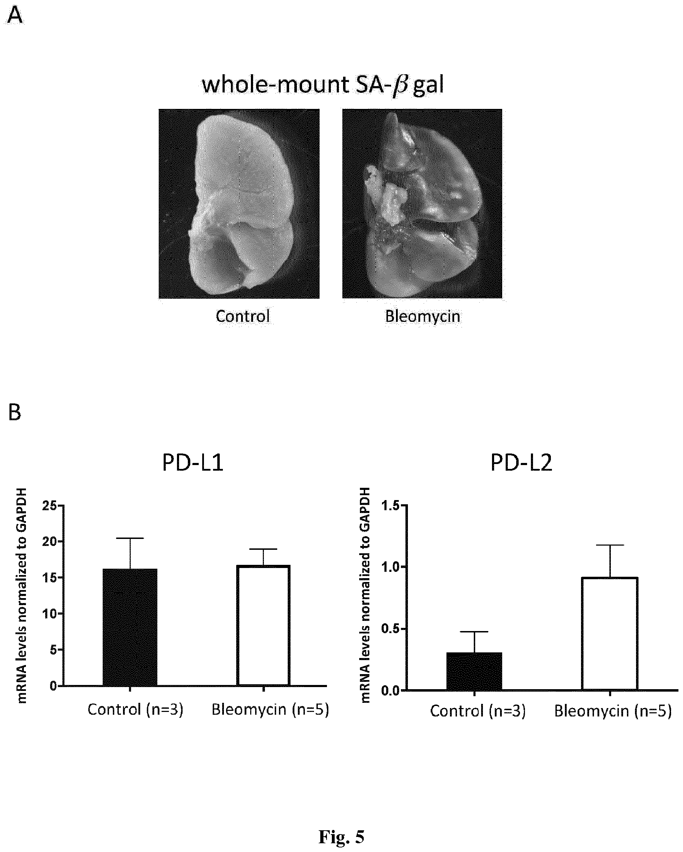

**Fig. 5** illustrates that PD-L2 is up-regulated in lung fibrosis. (A) Fibrosis was induced in C57BL6 mice lungs by intra-tracheal delivery of a single dose of bleomycin. Fourteen days post injury, SA-*β* galactosidase activity was analysed in whole organs. Senescent lungs (right image) presented the blue staining consequence of an increased SA-*β* galactosidase activity (B) PD-L1 and PD-L2 mRNA levels were evaluated by quantitative RT-PCR in lung tissue samples and normalised to the mRNA levels of glyceraldehyde-3-phosphate dehydrogenase (*GAPDH*). The data corresponds to the average +/- SD of three to five lungs. Statistical t-test analysis was performed to calculate significance (*P≤0.05).

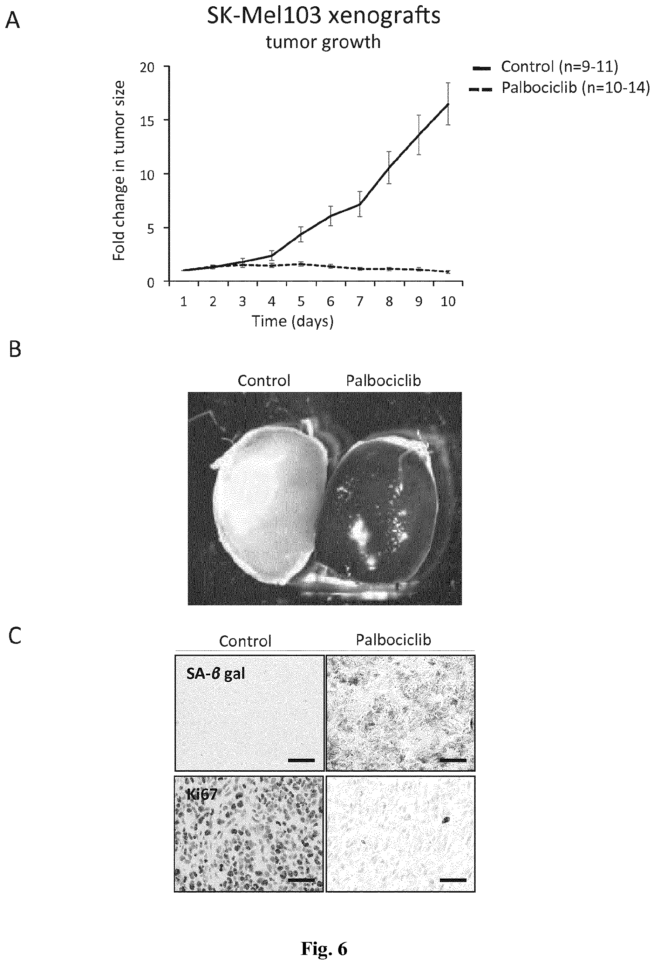

**Fig. 6** illustrates that SK-Mel-103 mouse xenografts halt growth and become senescent upon treatment with vehicle or palbociclib. (A) Human melanoma SK-Mel-103 cells were inoculated subcutaneously in immunedeficient nu/nu mice. When tumours had a volume of about 200 mm³, mice were treated with palbociclib by oral gavage and tumour progression was analysed by caliper measurement. The data represents the average +/- SEM of 9-11 control (continuous line) and 10-14 palbociclib-treated (dashed line) independent xenografts. (B) SA-*β* galactosidase activity was analysed in whole tumours. Senescent tumours (right) presented the blue staining consequence of an increased SA-*β* galactosidase activity. (C) SA-*β* galactosidase activity was analysed on tumour sections (upper images). Expression of the proliferation marker Ki67 was evaluated on the same tumours by immunohistochemistry (bottom images). Xenografts from palbociclib-treated mice presented the blue staining consequence of an increased SA-*β* galactosidase activity revealing the presence of senescent cells (upper right image).

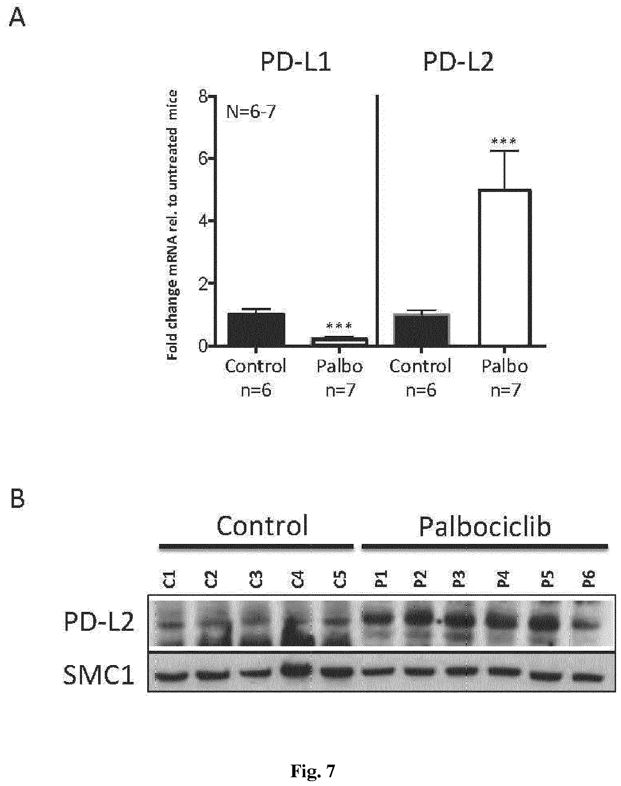

**Fig. 7** illustrates that senescent SK-Mel-103 mouse xenografts up-regulate PD-L2. (A) PD-L1 and PD-L2 mRNA levels were evaluated by quantitative RT-PCR of human melanoma SK-Mel-103 cells inoculated subcutaneously in immune-deficient nu/nu mice treated with either vehicle or palbociclib. Values were normalised to the housekeeping gene TATA-box binding protein (TBP) and represented relative to vehicle-treated tumours. The graphs depict the average +/- SD of six to seven independent xenografts. Statistical t-test analysis was performed to calculate significance (****P*≤0.001). (B) PD-L2 levels were analysed in vehicle and palbociclib treated tumours by Western blotting. The protein levels of anti-Structural Maintenance of Chromosomes (anti-SMC1) were used as loading control.

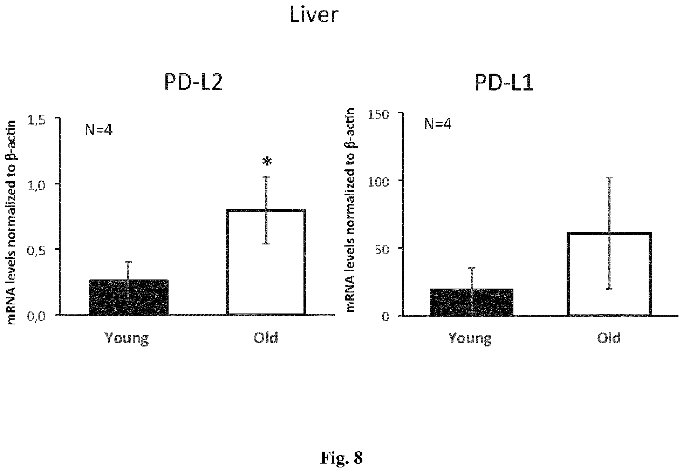

**Fig. 8** illustrates PD-L2 upregulation in aged individuals. PD-L2 (left) and PD-L1 (right) mRNA levels were evaluated by quantitative RT-PCR in livers from young mice (10 weeks of age) (grey bars) and old mice (75 weeks of age) (white bars). Values were normalised to the housekeeping gene *β-actin.* The graphs represent the average +/- SD of four mice. Statistical t-test analysis was performed to calculate significance (*P≤0.05).

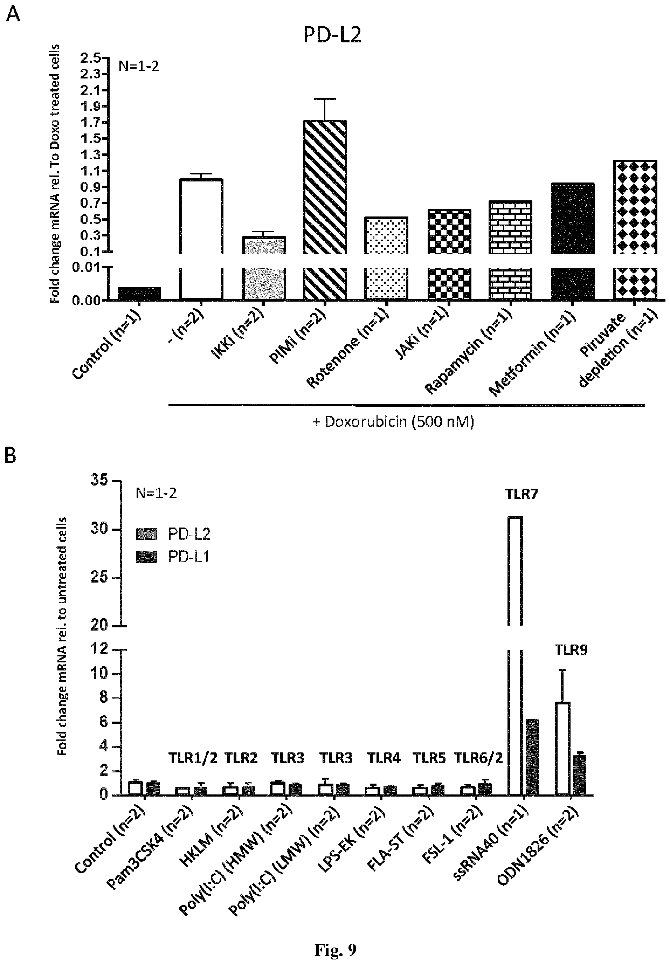

**Fig. 9** illustrates the effect of enzymatic inhibitors and TLRs agonists in the transcriptional regulation of PD-L2 and PD-L1. (A) PD-L2 mRNA levels were evaluated by quantitative RT-PCR in C2C12 cells treated with 500 nM doxorubicin for 48 hours in the presence or absence of commercial inhibitors against enzymes known to be involved in senescence/SASP/DNA-damage responses: IKKi (50 µM), PIMi (1 µM), rotenone (1 µM), JAKi (3 µM), rapamycin (50 nM) and metformin (5 mM) or cultured in pyruvate-depleted media. (B) PD-L1 (white bars) and PD-L2 (black bars) mRNA levels were evaluated by quantitative RT-PCR in C2C12 cells incubated for 6 hours in the presence or absence the following TLRs agonists: TLR1/2 agonist (Pam3CSK4, 300 ng/ml), TLR2 agonist (HKLM, 10⁸ cells/ml), TLR3 agonist (Poly (I:C) HMW and LMW, 10 µg/ml), TLR4 agonist (LPS-EK, 10 ug/ml), TRL5 agonist (FLA-ST, 1 µg/ml ), TRL6/2 agonist (FSL-1, 100 ng/ml), TRL7 agonist (ssRNA40, 5 µg/ml) and TLR9 agonist (ODN1826, 5 µM). Data was normalised to the mRNA levels of the housekeeping gene *β-actin* and represented as relative to doxorubicin treated cells (A) or untreated cells (B). These experiments were performed either once or twice. In the second case, data represents the average +/- SD of two independent experiments.

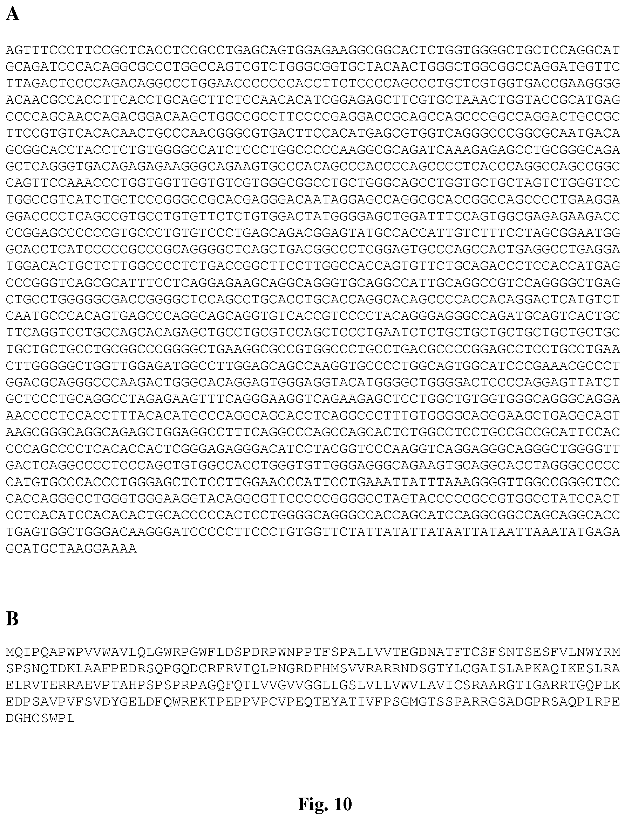

**Fig. 10** shows the DNA sequence (A) and the protein sequence (B) of Programmed Cell Death Protein 1 (PD-1).

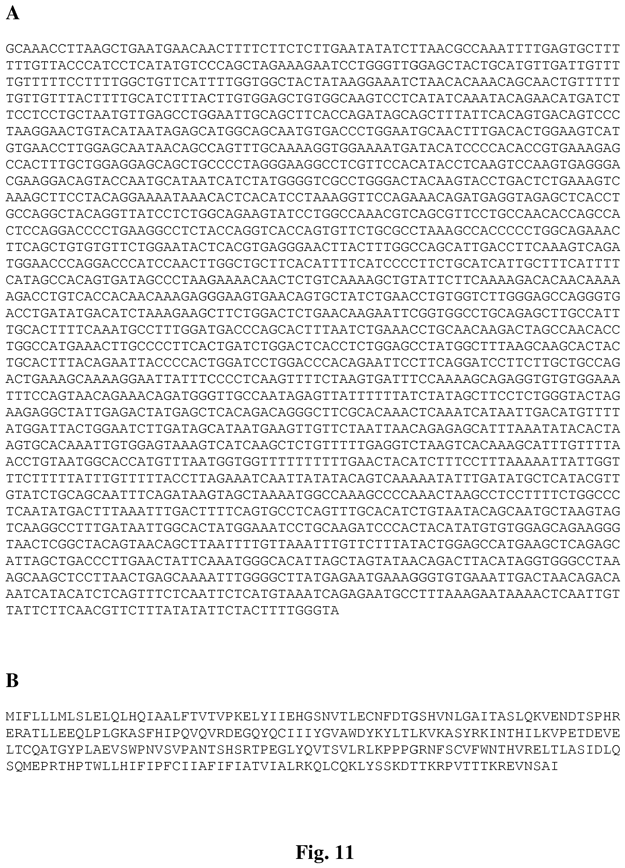

**Fig. 11** shows the DNA sequence (A) and the protein sequence (B) of Programmed Death Ligand 2 (PD-L2).

### DETAILED DESCRIPTION OF THE INVENTION

As defined above, the present invention is based in the unexpected finding that the expression of the products of the gene of PD-L2 (both the mRNA and the protein) is upregulated in damaged and/or senescent cells. PD-L2 upregulation seems to result in an increase of the signalling coming mediated by its known receptor, PD-1, so that damaged and/or senescent cells induce an immunosuppressive environment which favours that said cells are not eliminated by the immune system. This is a very important finding with applicability for a number of methods and/or uses, many of which are set forth herein as aspects of the present invention.

A first application is that PD-L2 upregulation can be used to identify damaged and/or senescent cells and/or to detect the presence of damaged and/or senescent cells in a sample. As it has been discussed previously, there is currently no established method for the identification of damaged and/or senescent cells, the lack of specificity being one of the main problems. Observing or determining PD-L2 upregulation in a cell or sample means an unprecedented method for that purpose, that implies an increase of specificity over the methods that rely in the upregulation of proteins such as p16 or p21, since PD-L2 expression in basal/normal conditions is usually low and restricted to limited types of cells.

Both the method for identifying damaged and/or senescent cells and the method of detecting the presence of damaged and/or senescent cells in a sample are both based in the same principle: detecting the expression of a product of the PD-L2 gene (which gene is usually denominated *PDCD1LG2*) and observing an increase in the level of said product with regard to a reference value. Thus, in principle, the method for identifying damaged and/or senescent cells can be considered a specific embodiment of the method for detecting the presence of damaged and/or senescent cells in a sample, wherein the cells of the sample maintain their integrity throughout the performance of the method and can be observed or treated as individual entities, identifiable with regard to other cells present in the same sample (for instance, due to their position in the sample, as could be the case in the analysis of cell cultures, tissue samples or cells fixed to any kind of support such as, for instance, a membrane or a polymeric support). But, taking into account that one of the most frequent embodiments of the method of identification of damaged and/or senescent cells will be its application to biological samples taken from one individual, the high increases in the levels of PD-L2 in damaged and/or senescent cells observed by the present inventors with regard to control cells and that the identification of particular cells of biological samples where a particular event has happened is usually done visually, by the detection of the presence of marker (such as a colorimetric or fluorimetric signal) that is absent in other cells also present in the same sample, so that the reference value can be considered the absence of signal in surrounding cells, it has been considered clearer to set forth both methods as different methods, although some features are common to both of them.

The detection of the presence of damaged and/or senescent cells in a sample from the level of the PD-L2 mRNA can be done by qRT-PCR, as in some assays of Examples of the present application. In that case, the reference value can be the PD-L2 mRNA level detected in a control sample which is considered not to contain damaged/senescent cells that is submitted to the same steps that the assayed sample, as in the assays shown in the present application, which control sample should preferable be selected so that it shares as many features as possible with the assayed sample other that the presence of damaged/senescent cells, for instance, same tissue, extracted from a subject of the same age or sex, a sample taken from the same individual but extracted from a tissue or a part of organ where the presence of damaged/senescent cells has been previously discarded. But the reference value can also be a value previously obtained, for instance as the average value of a previous study of samples where a number of control samples has been analyzed; this latter alternative could be of particular use in clinical practice. An increase of at least 2 fold, most preferably, at least 6 fold or higher, is preferable, in order to have more certainty in conclude that damaged and/or senescent cells are present in the assayed sample.

When the expression of PD-L2 protein is determined, given the basal low level of expression of PD-L2 protein, a Western blot and the visual identification of the corresponding band might be considered enough to decide that damaged/senescent cells were present in the assayed sample. Preferably, a control sample will have been included in the same assayed and it will be concluded that damaged/senescent cells are present in the assayed sample when the signal corresponding to the assayed sample is higher than the signal corresponding to the control sample (which signal in the control sample can have a value of 0, because its intensity might be not high enough to be detected).

The determination by Western blot can be considered a particular embodiment of the methods wherein molecules that bind to PD-L2 mRNA or protein are added to the assayed sampled and the binding of said molecule is determined in order to decide if damaged/senescent cells are present in the assayed sample and/or to identify a cell as a damaged/senescent cell.

Preferable, the molecule that binds to PD-L2 mRNA or PD-L2 protein will be labelled, that is, it will contain, as a part of the molecule, a chemical group, residue or moiety, whose presence will be easy to detect and quantify; typical examples of such molecules are those with a moiety that can be visualized by a coloured signal detectable in the range of frequencies of visual light, or those that emit a signal in frequency after being excited with light of a different frequency, as happened with fluorophores. Another possibility is that the molecule is selected so that its presence can be determined by the addition of a second molecule that is labelled or that can give rise to a reaction where a molecule easy to detect appears or disappears. In that sense, immunoassays where a primary antibody is used and its binding is revealed by a secondary antibody liked to a label such as a fluorophore or to an enzyme which can give rise to a coloured molecule, such as horseradish peroxidase or alkaline phosphatase, are well known by those skilled in the art and be easily adapted both to immunohistochemical assays or ELISA.

The molecule that binds to PD-L2 mRNA or PD-L2 protein will be selected in accordance to the product of the PD-L2 gene to detect and, preferably, taken into account the species of the subject. If the molecule is intended to bind PD-L2 mRNA, the molecule to add can be a molecule complementary to said mRNA, preferably carefully selected so that the binding is specific. For instance, the added molecule can be an oligonucleotide complementary to a fragment of said mRNA and capable to bind to it, including natural siRNAs and analogues thereof chemically modified to increase the binding or to facilitate the entrance into the cells. Those skilled in the art can easily design such oligonucleotides and analogues thereof from the cDNA sequences, that are available in public databases, such as the reference sequences NM_025239.3 (SEQ ID NO:3) and NM_021396.2 (SEQ ID NO:25) mentioned below and that correspond to *Homo sapiens* and *Mus musculus* PD-L2 cDNA, respectively.

In principle, both the method of determination of the presence of damaged and/or senescent cells and the method of identification of damaged/senescent cells are compatible with a broad meaning of the concept of "assayed sample", that includes not only biological samples taken from a subject (such as biopsies or samples of biological fluids such as blood, urine, saliva or even cerebrospinal fluid) but also whole organisms, even living beings. This is an advantage with regard to methods such as the one of the determination of β-gal signal. But carrying out the determination of the presence of damaged and/or senescent cells in whole living animals preferably implies to provide means for easily detecting a signal associated to PD-L2 presence/level, such as imaging it, and determining the possible presence of damaged/senescent cells from such images. That will usually involve administering to the living organism at least a molecule capable to bind to PD-L2 mRNA or PD-L2 protein, which molecule emits a signal that is easy to transform in an image that can be used for the determination or, in small animals, a signal that can be visualized easily. Thus, preferably, for human beings and other mammals, especially for clinical or veterinary diagnostic purposes, the concept of "sample" will be restricted to samples previously obtained, taken or extracted from the body of the subject (the human or other mammal), such as biopsies or samples of physiological fluids such as blood, saliva, urine, lacrimal secretion, cerebrospinal fluid or the like, cell obtained from said samples, cultures of said cells, cultures of established cell lines or cells individualized form any of said samples. In the case of animal models used for research purposes, the detection of the presence of damaged/senescent cells (or the identification of said cells) in a whole animal might be particularly useful, specially once the animal is dead, so that the variant of the method of the present invention of determining the presence of damaged and/or senescent cells that is carried out in a whole animal model, especially after its death, can be considered an embodiment of the present invention.

In order to be able to identify damaged and/or senescent cells, the molecule that binds to PD-L2 mRNA or PD-L2 protein may comprise a conventional contrast material that can be used to enhance the contrast of structures or fluids within the body during medical imaging. Such a material may be visible using Magnetic Resonance Imaging (MRI), Computed Tomography (CT) and/or Optical Imaging (OI).

Careful selection of the material used for the contrast material enables the molecule to be used in either diagnosis and/or therapy of various diseases and conditions associated with the presence of damaged and/or senescent cells.

The contrast material, which is visible using MRI, CT or OI, may comprise a metallic or non-metallic material. The contrast material may comprise a magnetic or non-magnetic material.

In embodiments where the contrast material is magnetic, it may comprise an MRI contrast material. The contrast material may comprise a paramagnetic or superparamagnetic material. For example, the contrast material may comprise iron, nickel, cobalt or dysprosium or a compound, such as oxide or alloy, which contain one or more of these elements. Preferably, the contrast material comprises magnetite (Fe₃O₄).

In embodiments where the contrast material is non-magnetic, it may comprise a MRI, CT or OI contrast material. For example, the contrast material may comprise gadolinium, gold, iodine or boro-sulphate. Each of these materials may be used as either MRI, CT or OI contrast materials. Preferably, the contrast material comprises gadolinium.

The molecule that binds to PD-L2 mRNA or PD-L2 protein might be a biomolecule (that is, a molecule that can be naturally found in a living being) or an analogue thereof that comprises one or more artificially done modifications with regard to the natural molecule, while maintaining, partially or completely, its biological activity. The molecule, biomolecule of analogue thereof may comprise a protein, peptide, antibody, chemical compound, genetic material, small molecule, drug or RNAi reagent. Preferably, the biomolecule is an antibody or an antigen-binding fragment thereof that specifically binds to PD-L2 protein, which antibody or fragment thereof comprises any kind of label, as defined above, that facilitates its detection, such as a contrast material as referred to above. Alternatively to the labelling part, or additionally to it, the molecule can comprise a part that facilitates its isolation because it binds to a second added molecule, for instance by affinity chromatography, or thanks to a property of the second added molecule, as happens with the magnetic beads.

Preferably, the contrast material is covalently linked to the biomolecule.

Additionally, it is compatible with both the method of determination of the presence of damaged and/or senescent cells and the method of identification of said cells, following an approach similar to those described by van Duersen group in 2016 (see reference *supra*) of determination based on p16: that the molecule whose level is determined is not directly PD-L2 mRNA or PD-L2 protein, but a mRNA or a protein expressed from a gene operatively linked to a PD-L2 promoter. The construct will usually be part of an expression vector or, in the case of animal models for research, can also be integrated in the genome. The expression vector can be directly administered to a particular organ of interest, or can be designed so that it is preferentially address to a particular tissue. This approach is particularly useful when the protein expressed from the gene is a reporter protein with a feature that facilitates its detection, such as light emission upon excitation, like the green fluorescent protein (GFP) or the like. Such approach also allows the expression of fused proteins, where the additional part of the protein has a different feature with facilitates additional assays or its purification, as happens with tagged proteins with a tail.

The method for the detection of damaged and/or senescent cells (or the method of identification of damaged and/or senescent cells) can be of use in the prognosis of a disease or condition characterised by the presence of damaged and/or senescent cells. The detection of the damaged and/or senescent cells will be done as it has been explained above, that is, by the method of the invention, so that the method can be also set forth as a method for the prognostic/diagnostic of a disease associated of damaged and/or senescent cells, wherein the presence of damaged and/or senescent cells is determined by the method of detection or the method of identification of said cells of the present invention. Preferably, as discussed above, the samples where the prognostic or diagnostic method is carried out will be restricted to samples previously obtained, taken or extracted from the body of the subject where the disease is to be diagnosed or prognosed, such as biopsies or samples of physiological fluids such as blood, saliva, urine, lacrimal secretion, cerebrospinal fluid or the like or cells obtained from said samples or processed samples obtained from the samples originally taken from the subject. The subject will be preferably a human or another mammal.

Additionally to the detection and prognosis methods that have been discussed in the previous paragraphs, the findings disclosed in the present application have applicability in other different aspects, that have been proposed as additional aspect of the same invention, which are based in the same inventive concept.

Interaction of PD-L2 and PD-1 has been found to provide a crucial negative co-stimulatory signal to T cells and other immune cells. Thus, there are herein provided methods of administering antagonists of the PD-L2/PD-1 interaction to eliminate damaged and/or senescent cells. Antagonists of the interaction between PD-L2 and PD-1 can be biomolecules (for example antibodies or peptides) that specifically bind to PD-L2 and/or PD-1 and inhibit/block/suppress the ability of PD-L2 to interact with or bind to PD-1. As a decrease in the expression of PD-1 or, preferably, PD-L2, will also decrease PD-1/PD-L2 interaction and the level of the biological activity of PD-L2, also encompassed within the definition of antagonists, as used herein, are those compounds that are capable to diminish PD-1 and/or PD-L2 expression, such as chemical compounds, drugs, small molecules and RNA interference reagents like microRNAs or siRNAs, analogs thereof, and even genetic material.

As a consequence, the antagonists of the present invention increase immune responses to damaged and/or senescent cells. As described herein, the inventors conclude that subversion of immune pathways that normally serve to temper T cell mediated immune responses and control autoimmunity, provide a mechanism by which damaged and/or senescent cells are able to evade a host's immune responses. Therefore, they propose that interfering in such mechanism will be of use for treating, ameliorating or preventing a disease associated to the presence of damaged and/or senescent cells. Thus, it can be said that the method of administering antagonists of the PD-L2/PD-1 interaction has, as a possible embodiment, a method for treating, ameliorating or preventing a disease associated to the presence of damaged and/or senescent cells wherein a therapeutically affective amount of an antagonist of the PD-L2/PD-1 interaction is administered to a subject in need thereof. It can be also said that the present invention provides an antagonist of the PD-L2/PD-1 interaction for treating, ameliorating or preventing a disease associated to the presence of damaged and/or senescent cells.

Agonists turn on a single cellular response by binding to a receptor, thus initiating a biochemical mechanism for change within a cell. Antagonists turn off that response by inhibiting/blocking/suppressing the interaction of the receptor with the agonist. In a broader sense, inhibitors of the expression of the ligand or the receptor can be also considered antagonists, because they inhibit/supress the interaction of the receptor with the ligand that acts as agonist.

In some embodiments, the antagonist of the PD-L2/PD-1 interaction is an antibody or an antigen-binding fragment thereof that specifically binds PD-L2 and/or PD-1.

As stated above, approved antibodies against PD-1 are already in clinical use for the treatment of cancer. The present inventors propose, as a possible embodiment of the present invention, to repurpose these anti-PD-1 antibodies to treat other diseases and conditions that are associated with damaged and/or senescent cells, including aging.

The terms "inhibit", "block" and "suppress" are used interchangeably herein to refer to any statistically significant decrease in biological activity, including full blocking of the activity. For example, "inhibition" can refer to a decrease of at least 10%, or at least 20%, or at least 30%, or at least 40%, or at least 50%, or at least 60%, or at least 70%, or at least 80%, or at least 90%, or about 100% in biological activity.

Accordingly, when the terms "inhibition", "blocking" or "suppression" are applied to describe for example, an effect on PD-L2 and/or PD-1 activity on T cell activation and/or T cell-mediated, directly or indirectly, cytolytic activity, the term refers to, for example, the ability of an antagonist such as, an anti-PD-L2 antibody and/or anti-PD-1 antibody, to statistically significantly decrease the activity of the antigen to which the antagonist binds. For example, the term inhibit, block or suppress may be used to refer to the ability of an anti-PD-L2 antibody and/or an anti-PD-1 antibody to decrease the activity of PD-L2 or PD-1 and/or the ability of the antibody to increase T cell mediated cytolytic activity *vitro in* or *in vivo,* relative to expression and/or T cell mediated cytolytic activity in an untreated cell population (i.e. control). The term inhibit, block or supress is also used herein to refer to the ability of an antagonist (e.g. anti-PD-L2 or anti-PD-1 antibody or antigen-binding fragment thereof) to decrease the ability of PD-L2 to interact with (i.e. bind to) PD-1.

A blocking antibody, an antagonist or a blocking agent is one which inhibits or reduces biological activity of the antigen it binds to, e.g. inhibiting or reducing the ability of PD-L2 to interact with or bind to PD-1. In certain embodiments blocking antibodies or antagonist antibodies substantially or completely inhibit the biological activity of the antigen. Desirably, the biological activity is reduced by at least 10%, 20%, 30%, 50%, 70%, 80%, 90%, 95%, or about 100%.

The term "antibody" as used in the present context means an immunoglobulin molecule that recognizes and specifically binds to a target, such as a protein, polypeptide, peptide, carbohydrate, polynucleotide, lipid or combination of the foregoing through at least one antigen recognition site within the variable region of the immunoglobulin molecule. As used herein, the term "antibody" encompasses intact polyclonal antibodies, intact monoclonal antibodies, antibody fragments, such as Fab, Fab', F(ab;)2, and Fv fragments, single chain mutants, multispecific antibodies such as bispecific antibodies generated from at least two intact antibodies, chimeric antibodies, humanized antibodies, human antibodies, fusion proteins comprising an antigen determination portion of an antibody, and any other modified immunoglobulin molecule comprising an antigen recognition site so long as that antibody exhibits the desired biological activity. Thus, in one embodiment, the antibody can be of any of the five major classes of immunoglobulins: IgA, IgD, IgE, IgG and IgM, or subclasses (isotypes) thereof (e.g. IgG1, IgG2, IgG3, IgG4, IgA1 and IgA2), based on the identity of their heavy-chain constant domains referred to as alpha, delta, epsilon, gamma, and mu, respectively. The different classes of immunoglobulin have different and well known subunit structures and three-dimensional configurations. Antibodies used in the invention can be naked or conjugated to other molecules such as toxins, radioisotopes, to form Antibody Drug Conjugates (ADC).

The terms "antibody" or "immunoglobulin" are used interchangeably herein, and include whole antibodies and any antigen binding fragment or single chains thereof. A typical whole antibody comprises at least two heavy (H) chains and two light (L) chains interconnected by disulphide bonds. Each heavy chain is comprised of a heavy chain variable region (abbreviated herein as VH) and a heavy chain constant region. The heavy chain constant region is comprised of three domains, CH1, CH2 and CH3. Each light chain is comprised of a light chain variable region (abbreviated as VL) and a light chain constant region. The light chain constant region is comprised of one domain, CL. The VH and VL regions can be further subdivided into regions of hypervariability, termed Complementary Determining Regions (CDR), interspersed with regions that are more conserved, termed framework regions (FW). Each VH and VL is composed of three CDRs and four FWs, arranged from amino-terminus to carboxy-terminus in the following order: FW1, CDR1, FW2, CDR2, FW3, CDR3, FW4. The variable regions of the heavy and light chains contain a binding domain that interacts with an antigen. The constant regions of the antibodies can mediate the binding of the immunoglobulin to host tissues and factors, including various cells of the immune system (e.g. effector cells) and the first component (Clq) of the classical complement system. Exemplary antibodies of the present disclosure include typical antibodies, scFvs, and combinations thereof where, for example, an scFv is covalently linked (for example, via peptidic bonds or via a chemical linker) to the N-terminus of either the heavy chain and/or the light chain of a typical antibody, or intercalated in the heavy chain and/or the light chain of a typical antibody. Additional exemplary antibodies herein include fusion proteins comprising an antibody portion, and any other modified immunoglobulin molecule comprising an antigen recognition site. For the purpose of this disclosure, the term antibody also encompasses Fc fusion proteins containing immunoglobulin-derived naturally occurring and/or synthetic amino acid sequences (e.g. peptibodies) that bind an expressed peptide on a cell of interest to be targeted (e.g. PD-L2).

The phrase "antigen binding fragment" refers to a portion of an intact antibody and/or refers to the antigenic determining variable regions of an intact antibody. It is known that the antigen binding function of an antibody can be performed by fragments of a full-length antibody. Examples of antibody fragments include, but are not limited to, Fab, Fab', F(ab')2 and Fv fragments, linear antibodies, single chain antibodies, diabodies, and multispecific antibodies formed from antibody fragments.

In particular embodiments, the antibodies used according to the disclosed methods have reduced effector function. In some embodiments, the antibodies contain mutations in the Fc region responsible for effector function.

Preferably, the antagonist is an anti-PD-L2 antibody or antigen binding fragment thereof.

Compared to PD-L1, constitutive basal expression of PD-L2 is low and it was initially believed to be restricted to professional APCs such as macrophages and dendritic cells, although in recent years it has been found that PD-L2 is also expressed in a variety of non-professional APCs including keratinocytes, endothelial cells, astrocytes, fibroblast and oligodendrocytes (Rozali *et al.,* 2012). As a result, no therapeutic uses for anti-PD-L2 antibodies have previously been described.

Interestingly, the relative affinity of PD-L2 for PD-1 was calculated to be 2-6 fold higher than that of PD-L1 for PD-1 (Youngnak *et al.,* 2003).

Moreover, due to the fact that, as alluded to above, compared to PD-L1, constitutive basal expression of PD-L2 is low and restricted to fewer cellular types (Rozali et al., 2012), the inventors anticipate that targeting of PD-L2 is more effective and has far less side effects than targeting either PD-L1 or PD-1.

The present invention, therefore, offers an unprecedented non-cytotoxic solution for the elimination of damaged and/or senescent cells and provides a therapeutic indication for antagonists of the PD-L2/PD-1 interaction, particularly anti-PD-L2 antibodies: to break the immunotolerance that prevents the elimination of senescent and/or damaged cells by the immune system in multiple human diseases.

Other antagonists of the PD-L2/PD-1 interaction can be readily identified and prepared using techniques known in the art and then used for eliminating damaged and/or senescent cells now that the inventors have shown that such cells upregulate their expression of PD-L2.

Other antagonists that could be used in the present invention include proteins, peptides, chemical compounds, genetic material, small molecules, drugs or RNAi reagents; anything that is able to disrupt or prevent the ligand/receptor interaction.

Methods of confirming that antagonists can inhibit the interaction of PD-L2 and PD-1 are also known.

When the antagonists are antibodies, previously known antibodies against PDl-1 or preferably PD-L2, particularly commercially available antibodies, can be used, provided that it is previously confirmed that the inhibit the interaction of PD-L2 and PD-1. Preferably, newly obtained antibodies will be used.

The methods for obtaining antibodies in general, and monoclonal antibodies in particular, are well known for those skilled in the art. Antibodies against PD-1 or, preferably, PD-L2, can be raised according to known methods, such as those mentioned in classic laboratory manuals as "Antibodies: A Laboratory Manual, Second edition", edited by E.A. Greenfield in 2014, by administering PD-1 or PD-L2 whole protein or a fragment or epitope thereof to a host animal which is a different from the mammal where the therapeutic effect is sought. Monoclonal antibodies in particular can be prepared and isolated by any technique that provides for the production of antibody molecules by continuous cell lines in culture, such as the hybridoma technique originally described by Kohler and Milstein (1975), the human B-cell hybridoma technique (Cote *et al.,* 1983), or the EBV-hybridoma technique (Cole *et al.,* 1985). Alternatively, as commented above, Fab and/or scFv expression libraries can be constructed to allow rapid identification of fragments having the desired specificity to the PD-1 receptor or PD-L2 ligand.

For the design of antibodies with a particular specificity, it is advantageous to resource to the annotated sequence of the protein or the DNA coding sequence, which facilitates, for instance, the choice as immunogen of a particular domain or region of the antigen to be targeted before generating the antibodies. NCBI Reference Sequence (NC_000007.14, Homo sapiens annotation release: 107, which is the current release on 29 September 2015) or UniProtKB P16671, in order to choose as immunogen, if wished, a particular domain or region of the antibody to be targeted or mutated before generating the antibodies.

The DNA sequence (2115 base pairs) corresponding to the mRNA of human Programmed Cell Death Protein 1 (PD-1) (GenBank accession no. NM 005018, version NM_005018.1) is provided in SEQ ID NO:1 and is also shown in Fig. 10A.

The protein sequence (288 amino acids) of human Programmed Cell Death Protein 1 (PD-1) is shown in SEQ ID NO:2 and Fig. 10B, and can be also found using NCBI accession no. NP 005009, version NP_005009.1).

The DNA sequence (2418 base pairs) of human Programmed Death Ligand 2 (PD-L2) (GenBank accession no. NM_025239, version NM_025239.2) is provided in SEQ ID NO:3 and is also shown in Fig. 11A.

The protein sequence (288 amino acids) of or human Programmed Death Ligand 2 (PD-L2) or human Programmed Cell Death 1 ligand 2 (PDCD1LG2) is shown in SEQ ID NO:4 and in Fig. 11B, and can be also found using NCBI accession no. NP 0079515, version NP_079515.2.

Antibodies that are intended for use in human beings can have been generated in a non-human immune system, such as in mice, but humanization can be necessary to enable their administration to human beings, in order to avoid adverse reactions. Humanized antibodies are antibodies, usually monoclonal antibodies, initially generated in a non-human species and whose protein sequences have been modified to increase their similarity to antibody variants produced naturally in humans, so that minimal sequence derived from non-human immunoglobulins remain. Even after humanization, the amino acid sequence of humanized antibodies is partially distinct from antibodies occurring naturally in human beings. Several processes are known for those skilled in the art for antibody humanization, as it has been reviewed, for instance, by Almagro and Fransson (2008), including: humanizing through production of a mouse-human (mouse Fab spliced to human Fc) chimera, which chimera might be further humanized by selective alteration of the amino acid sequence of the Fab portion; insertion of one or more CDR segments of the "donor" (non-human antibody) by replacing the corresponding segments of a human antibody, which can be done using recombinant DNA techniques to create constructs capable of expression in mammalian cell culture, or even avoiding the use of non-human mammals by creating antibody gene libraries usually derived from human RNA isolated from peripheral blood and displayed by micro-organisms or viruses (as in phage display) or even cell free extracts (as in ribosome display), selection of the appropriate intermediate product (usually, antibody fragments such as Fab or scFv) and obtaining full antibodies for instance, again, recombinant DNA techniques. Several patent documents have been dedicated to humanization methods like, for instance US6054297, assigned to Genentech; US5225539 and US4816397 are also useful references.

Such antagonists may be suitable for use in treating any condition where it is desired to eliminate damaged and/or senescent cell, or to treat, ameliorate or prevent a disease characterised by the presence of damaged and/or senescent cells. Such diseases include, for example, degenerative diseases, type 2 diabetes, sarcopenia, cataracts, radiation-induced oral mucositis, brain and heart aneurysms, glaucoma, Alzheimer's disease, Parkinson's disease, cystic fibrosis, intestinal bowl disease, osteoarthritis and inverted disc degeneration.

Other diseases and conditions, in addition to those recited above, that could be treated, ameliorated or prevented (specially after their prognosis or diagnosis, as described above) by the present invention include the following.

Wiedemann-Rautenstrauch syndrome of neonatal progeria, the Werner syndrome of adult progeria, Hutchinson-Gilford syndrome, Rothmund Thompson syndrome, Mulvill-Smith syndrome, Cockayne syndrome, Dyskeratosis Congenita, idiopathic pulmonary fibrosis, liver fibrosis, renal fibrosis, oral mucous fibrosis, cardiac fibrosis, pancreatic fibrosis, cancer, aplastic anaemia, emphysema, degeneration of cartilage, obesity, macular degeneration, glaucoma, pulmonary hypertension, chronic obstructive pulmonary disease, renal transplantation. Fibrotic diseases are an example of degenerative diseases characterized by the abnormal deposition of extracellular matrix proteins, most notably collagen, that obliterate the architecture and function of the underlying organ or tissue. These diseases affect a large number of people but presently no current treatment has proven to be effective in preventing their progression.

Fibrosis can occur in many tissues within the body, typically as a result of inflammation or damage. Examples of fibrotic diseases are systemic sclerosis, pulmonary fibrosis, kidney fibrosis, heart fibrosis and liver fibrosis, brain glial scar and atherosclerosis. The accumulation of senescent cells is a common event in fibrotic pathologies. By mediation of their secreted factors (SASP), senescent cells cause chronic inflammation that is largely responsible for the symptoms and progression of these pathologies. Consequently, targeting senescent cells offers the potential of a promising therapeutic approach for fibrotic diseases.

Damaged and/or senescent cells are also present in benign lesions such as nevi and skin scars post-wound healing. The present invention also relates to cosmetic improvements in skin, for example, the removal of moles, which contain melanocytic nevi that have undergone senescence. Thus, also an additional aspect of the invention is a method for cosmetic improvement of the skin, including the removal of moles from the skin of a subject, which method comprises administering to the subject an antagonist of the PD-L2/PD-1 interaction.

Damaged and/or senescent cells also accumulate in aged organisms and negatively influence lifespan; thus, the use of the antagonist of the PD-L2/PD-1 interaction can have a double effect with regard to ageing: a therapeutic effect, mainly with regard to diseases, disorders or conditions closely associated to ageing, such as degenerative diseases, an also a non-therapeutic effect, related to the increase of lifespan, reducing biological ageing and/or the amelioration or removal of ageing effects such as aesthetic effects or mobility that do not reach a pathological level. The present invention can, therefore, also be used for the alleviation of the side effects of and/or accelerated ageing caused by treatment with chemotherapy or radiotherapy, which inflicts damage on non-cancerous, healthy tissue and drives cellular senescence in these tissues, which play a pathological role in these side effects. The invention also encompasses the use of PD-L2/PD-1 antagonists in combination with other compounds, treatments or agents that are known to drive cellular damage or senescence, including any kind of radiation, both the non-medical radiation and the "medical" radiation applied with a medical purpose (such as a diagnostic objective, as X-rays, or a therapeutic objective, as radiotherapy applied in cancer or in some skin disorders). Therefore, is also an aspect of the invention an antagonist of the PD-L2/PD-1 interaction for use in combination with chemotherapy, radiotherapy and/or any other drugs, treatments or agents that promote damaged and/or senescent cells, such as anti-retroviral drugs, corticoids, PPAR gamma agonists and/or exposure to radiation non-intended for cancer therapy. As the term is used herein, "combination" comprises the simultaneous administration of the drug, treatment or agent together with the antagonist of the PD-L2/PD-1 interaction, but also the administration of the antagonist of the PD-L2/PD-1 interaction subsequently.

Damaged and/or senescent cells are also produced in response to cancer therapy-induced genotoxic stress produce by, for example, standard radiotherapy and chemotherapy used to treat cancer, and have been shown to have a negative impact on tumour regression and a detrimental effect on the health of cancer survivors. Chemotherapeutic drugs, such as palbociclib, ribociclib and abemaciclib, which are selective inhibitors against CDK4 and CDK6 kinases, induce a senescent response in most tumor cells. Moreover, additional senescence-inducing chemotherapies are likely to be developed in the future. The present invention can, therefore, also be used for the treatment of cancer, and in particular the selective elimination of residual cancer cells which have survived prior chemotherapy treatment. Residual cancer cells that have survived prior chemotherapy treatment may experience elevated *β*-galactosidase and/or α fucosidase activity, as a result of DNA damage that triggers the cellular senescence program.

The present invention can, therefore, be used in relation to the above diseases and conditions, and could even be used in combination with chemotherapies in order to increase their therapeutic efficacy.

The present invention can also be used for the removal of senescent cells following treatment with anti-retroviral drugs, which can cause subcutaneous lipolysis, metabolic dysfunction, and accelerated ageing. Examples of these antiretroviral drugs may include abacavir, azacytidine, azidothymidine (AZT), dideoxycytidine, efavirenz, invirase, ixabepilone, lamivudine (3TC), lopinavir, nevirapine, ritonavir, stavudine, tenofovir, tipranavir, or thiotepa.

The present invention can also be used for the removal of senescent cells following treatment with corticoids including cortisol, prednisone, prednisolone, dexamethasone, triamcinolone, beclometasone, fludrocortisone, deoxycorticosterone, and aldosterone. Treatment with these corticoids can lead to cellular senescence and tissue degeneration, for example in tendons following dexamethasone administration, metabolic dysfunction such as obesity and insulin resistance.

The present invention can also be used for the removal of senescent cells following long term use of PPAR gamma agonists, including thiazolidinediones such as rosiglitazone, pioglitazone, and troglitazone. The removal of senescent cells will prevent the induction of the SASP, which may be associated with increased adverse cardiac events, and increased risk of cancer.

The present invention can also be used for the treatment of cellular hypertrophy, including cardiac hypertrophy. Senescent cells retain growth factor signaling, but being unable to undergo division, continue to enlarge and undergo hypertrophy. Senescent cells frequently experience constitutive mammalian target of rapamycin (mTOR) complex activation.

The present invention can also be used for the treatment of chronic inflammation, caused by cytokines released as part of the SASP from senescent cells.

The present invention also relates to reducing the side effects of an accelerated ageing caused by exposure to radiation (both medical and non-medical radiation), which causes DNA damage and cellular senescence, with subsequent release of SASP cytokines. Senescence may be caused by various forms of radiation including alpha radiation, beta radiation, gamma radiation. Examples include exposure to medical x-rays, PET scans, CT scans; nuclear radiation such as from nuclear fallout or nuclear accidents, UV radiation from sun exposure or other UV sources, cosmic radiation from extended time at high altitudes (e.g. pilots and aeroplane attendants, frequent aeroplane travelers, astronauts), and medical radiotherapy.

Thus, it can be also said that, in an aspect of the invention, there is provided a method of eliminating damaged and/or senescent cells, the method comprising administering to a subject a therapeutically effective amount of an antagonist of the PD-L2/PD-1 interaction.

Also in another aspect of the invention, there is provided a method of treating, ameliorating or preventing a disease or condition characterized by the presence of damaged and/or senescent cells, the method comprising administering to a subject in need thereof such treatment, a therapeutically effective amount of an antagonist of the PD-L2/PD-1 interaction.

It will be appreciated that antagonists according to the invention may be used in a medicament, which may be a monotherapy (i.e. the sole use of that antagonist or medicament), for eliminating damaged and/or senescent cells or for treating, ameliorating or preventing a disease characterized by the presence of damaged and/or senescent cells. Alternatively, antagonists according to the invention may be used as an adjunct to, or in combination with, known therapies for eliminating damaged and/or senescent cells or for treating, ameliorating or preventing a disease characterized by the presence of damaged and/or senescent cells. For example, the antagonist may be used in combination with known agents for treating pulmonary fibrosis, such as corticosteroids. Similarly, the antagonists of the invention may be used in combination with known techniques for treating cancer, such as chemotherapy and/or radiotherapy.

There is no restriction on which antagonist as described herein should be administered to which patient. Rather, it is intended that any of the antagonist described herein can be administered to any patient as described herein. It is expressly intended by the inventors, in fact, that each and every combination of antagonist, and indicated patient group, is encompassed by this invention. The invention thus includes each and every possible combination of antagonist and indicated patient group.

The antagonists according to the invention may be combined in compositions having a number of different forms depending, in particular, on the manner in which the composition is to be used. Thus, for example, the composition may be in the form of a powder, tablet, capsule, liquid, ointment, cream, gel, hydrogel, aerosol, spray, micellar solution, transdermal patch, liposome suspension or any other suitable form that may be administered to a person or animal in need of treatment. It will be appreciated that the vehicle of medicaments according to the invention should be one which is well-tolerated by the subject to whom it is given.

Medicaments comprising antagonists according to the invention may be used in a number of ways. For instance, oral administration may be required, in which case the antagonists may be contained within a composition that may, for example, be ingested orally in the form of a tablet, capsule or liquid. Compositions comprising antagonists of the invention may be administered by inhalation (for example, intranasally). Compositions may also be formulated for topical use. For instance, creams or ointments may be applied to the skin, for example, adjacent the treatment site.

Antagonists according to the invention may also be incorporated within a slow- or delayed-release device. Such devices may, for example, be inserted on or under the skin, and the medicament may be released over weeks or even months. The device may be located at least adjacent the treatment site. Such devices may be particularly advantageous when long-term treatment with antagonists used according to the invention is required and which would normally require frequent administration (for example, at least daily injection).

In a preferred embodiment, antagonists and compositions according to the invention may be administered to a subject by injection into the blood stream or directly into a site requiring treatment. Injections may be intravenous (bolus or infusion) or subcutaneous (bolus or infusion), or intradermal (bolus or infusion).

It will be appreciated that the amount of antagonist that is required is determined by its biological activity and bioavailability, which in turn depends on the mode of administration, the physiochemical properties of antagonist, and whether it is being used as a monotherapy or in a combined therapy. The frequency of administration will also be influenced by the half-life of the antagonist within the subject being treated. Optimal dosages to be administered may be determined by those skilled in the art, and will vary with the particular antagonist in use, the strength of the pharmaceutical composition, the mode of administration, and the advancement of the disease or condition being treated. Additional factors depending on the particular subject being treated will result in a need to adjust dosages, including subject age, weight, gender, diet and time of administration.

Generally, a daily dose of between 0.001 µg/kg of body weight and 10 mg/kg of body weight of the antagonist according to the invention may be used for eliminating damaged and/or senescent cells or for treating, ameliorating or preventing a disease characterized by the presence of damaged and/or senescent cells, depending upon which antagonist is used. More preferably, the daily dose is between 0.01 (µg/kg of body weight and 1 mg/kg of body weight, more preferably between 0.1 (µg/kg and 100 µg/kg body weight, and most preferably between approximately 0.1 µg/kg and 10 µg/kg body weight.

The antagonists may be administered before, during or after onset of the condition or disease characterized by the presence of damaged and/or senescent cells. Daily doses may be given as a single administration (for example, a single daily injection). Alternatively, the antagonists may require administration twice or more times during a day. As an example, antagonists may be administered as two (or more, depending upon the severity of the bacterial infection being treated) daily doses of between 0.07 µg and 700 mg (i.e. assuming a body weight of 70 kg). A patient receiving treatment may take a first dose upon waking and then a second dose in the evening (if on a two-dose regime) or at 3- or 4-hourly intervals thereafter. Alternatively, a slow release device may be used to provide optimal doses of antagonists according to the invention to a patient without the need to administer repeated doses.

Known procedures, such as those conventionally employed by the pharmaceutical industry (for example, *in vivo* experimentation, clinical trials, etc.), may be used to form specific formulations comprising the antagonists according to the invention and precise therapeutic regimes (such as daily doses of the antagonists and the frequency of administration). The inventors believe that they are the first to describe a composition for treating disease characterized by the presence of damaged and/or senescent cells based on the use of the antagonists of the invention.

Hence, in another aspect of the invention, there is provided a damaged and/or senescent cells-treatment composition, comprising an antagonist of the PD-L2/PD-1 interaction and a pharmaceutically acceptable vehicle.

The phrase "damaged and/or senescent cells-treatment composition" can mean a pharmaceutical formulation used in the therapeutic amelioration, prevention or treatment of any disease or condition characterised by the presence of damaged and/or senescent cells in a subject.

The invention also provides, in an additional aspect, a process for making the damaged and/or senescent cells-treatment composition according to the fourth aspect, the process comprising contacting a therapeutically effective amount of an antagonist of the PD-L2/PD-1 interaction and a pharmaceutically acceptable vehicle.

A "therapeutically effective amount" of an antagonist of the invention is any amount which, when administered to a subject, is the amount of antibody that is needed to eliminate damaged and/or senescent cells, or produce the desired effect.

For example, the therapeutically effective amount of antagonist used may be from about 0.001 mg to about 1000 mg, and preferably from about 0.01 mg to about 500 mg. It is preferred that the amount of agent is an amount from about 0.1 mg to about 100 mg, and most preferably from about 0.5 mg to about 50 mg. As a guide, the dose of antibody used in the Examples described herein was 40 mg/kg.

A "pharmaceutically acceptable vehicle" as referred to herein, is any known compound or combination of known compounds that are known to those skilled in the art to be useful in formulating pharmaceutical compositions.

A "subject", as used herein, may be a vertebrate, mammal or domestic animal. Hence, compositions and medicaments according to the invention may be used to treat any mammal, for example livestock (for example, cattle), pets, or may be used in other veterinary applications. Most preferably, though, the subject is a human being.

In one embodiment, the pharmaceutically acceptable vehicle may be a solid, and the composition may be in the form of a powder or tablet. A solid pharmaceutically acceptable vehicle may include one or more substances which may also act as flavoring agents, lubricants, solubilisers, suspending agents, dyes, fillers, glidants, compression aids, inert binders, sweeteners, preservatives, dyes, coatings or tablet-disintegrating agents. The vehicle may also be an encapsulating material. In powders, the vehicle is a finely divided solid that is in admixture with the finely divided active agents according to the invention. In tablets, the active agent may be mixed with a vehicle having the necessary compression properties in suitable proportions and compacted in the shape and size desired. The powders and tablets preferably contain up to 99% of the active agents. Suitable solid vehicles include, for example calcium phosphate, magnesium stearate, talc, sugars, lactose, dextrin, starch, gelatin, cellulose, polyvinylpyrrolidine, low melting waxes and ion exchange resins. In another embodiment, the pharmaceutical vehicle may be a gel and the composition may be in the form of a cream or the like.

However, the pharmaceutical vehicle may be a liquid, and the pharmaceutical composition is in the form of a solution. Liquid vehicles are used in preparing solutions, suspensions, emulsions, syrups, elixirs and pressurized compositions. The active agent according to the invention may be dissolved or suspended in a pharmaceutically acceptable liquid vehicle such as water, an organic solvent, a mixture of both or pharmaceutically acceptable oils or fats. The liquid vehicle can contain other suitable pharmaceutical additives such as solubilisers, emulsifiers, buffers, preservatives, sweeteners, flavoring agents, suspending agents, thickening agents, colours, viscosity regulators, stabilisers or osmo-regulators. Suitable examples of liquid vehicles for oral and parenteral administration include water (partially containing additives as above, for example, cellulose derivatives, preferably sodium carboxymethyl cellulose solution), alcohols (including monohydric alcohols and polyhydric alcohols, for example, glycols) and their derivatives, and oils (for example, fractionated coconut oil and arachis oil). For parenteral administration, the vehicle can also be an oily ester such as ethyl oleate and isopropyl myristate. Sterile liquid vehicles are useful in sterile liquid form compositions for parenteral administration. The liquid vehicle for pressurised compositions can be a halogenated hydrocarbon or other pharmaceutically acceptable propellant.

Liquid pharmaceutical compositions, which are sterile solutions or suspensions, can be utilised by, for example, intramuscular, intrathecal, epidural, intraperitoneal, intravenous and particularly subcutaneous injection. The antagonist may be prepared as a sterile solid composition that may be dissolved or suspended at the time of administration using sterile water, saline or other appropriate sterile injectable medium.

The antagonists and compositions of the invention may be administered orally in the form of a sterile solution or suspension containing other solutes or suspending agents (for example, enough saline or glucose to make the solution isotonic), bile salts, acacia, gelatin, sorbitan monoleate, polysorbate 80 (oleate esters of sorbitol and its anhydrides copolymerized with ethylene oxide) and the like. The antagonists used according to the invention can also be administered orally either in liquid or solid composition form. Compositions suitable for oral administration include solid forms, such as pills, capsules, granules, tablets and powders, and liquid forms, such as solutions, syrups, elixirs and suspensions. Forms useful for parenteral administration include sterile solutions, emulsions and suspensions.

It will be appreciated that the invention extends to any nucleic acid or peptide or variant, derivative or analogue thereof, which comprises substantially the amino acid or nucleic acid sequences of any of the sequences referred to herein, including functional variants or functional fragments thereof. The terms "substantially the amino acid/nucleotide/peptide sequence", "functional variant" and "functional fragment", can be a sequence that has at least 40% sequence identity with the amino acid/nucleotide/peptide sequences of any one of the sequences referred to herein, for example 40% identity with the sequences referred to herein.

Amino acid/nucleotide/peptide sequences with a sequence identity which is greater than 50%, more preferably greater than 65%, 70%, 75%, and still more preferably greater than 80% sequence identity to any of the sequences referred to herein are also envisaged. Preferably, the amino acid/nucleotide/peptide sequence has at least 85% identity with any of the sequences referred to, more preferably at least 90%, 92%, 95%, 97%, 98%, and most preferably at least 99% identity with any of the sequences referred to herein.

The skilled technician will appreciate how to calculate the percentage identity between two amino acid/nucleotide/peptide sequences. In order to calculate the percentage identity between two amino acid/nucleotide/peptide sequences, an alignment of the two sequences must first be prepared, followed by calculation of the sequence identity value. The percentage identity for two sequences may take different values depending on: (i) the method used to align the sequences, for example, ClustalW, BLAST, FASTA, Smith-Waterman (implemented in different programs), or structural alignment from 3D comparison; and (ii) the parameters used by the alignment method, for example, local *versus* global alignment, the pair-score matrix used (for example, BLOSUM62, PAM250, Gonnet etc.) and gap-penalty, for example, functional form and constants.

Having made the alignment, there are many different ways of calculating percentage identity between the two sequences. For example, one may divide the number of identities by: (i) the length of shortest sequence; (ii) the length of alignment; (iii) the mean length of sequence; (iv) the number of non-gap positions; or (iv) the number of equivalenced positions excluding overhangs. Furthermore, it will be appreciated that percentage identity is also strongly length-dependent. Therefore, the shorter a pair of sequences is, the higher the sequence identity one may expect to occur by chance.

Hence, it will be appreciated that the accurate alignment of amino acid or nucleic acid sequences is a complex process. The popular multiple alignment program ClustalW (Thompson *et al.,* 1994) is a preferred way for generating multiple alignments of proteins or DNA in accordance with the invention. Suitable parameters for ClustalW may be as follows: For DNA alignments: Gap Open Penalty = 15.0, Gap Extension Penalty = 6.66, and Matrix = Identity. For protein alignments: Gap Open Penalty = 10.0, Gap Extension Penalty = 0.2, and Matrix = Gonnet. For DNA and Protein alignments: ENDGAP = -1, and GAPDIST = 4. Those skilled in the art will be aware that it may be necessary to vary these and other parameters for optimal sequence alignment.

Preferably, calculation of percentage identities between two amino acid/nucleotide/peptide sequences may then be calculated from such an alignment as (N/T)*100, where N is the number of positions at which the sequences share an identical residue, and T is the total number of positions compared including gaps but excluding overhangs. Hence, a most preferred method for calculating percentage identity between two sequences comprises (i) preparing a sequence alignment using the ClustalW program using a suitable set of parameters, for example, as set out above; and (ii) inserting the values of N and T into the following formula: Sequence Identity = (N/T)*100.

Alternative methods for identifying similar sequences will be known to those skilled in the art. For example, a substantially similar nucleotide sequence will be a sequence which hybridises to a nucleotide sequence encoding a peptide according to the first aspect, or a functional fragment or functional variant thereof, or their complements, under stringent conditions. By stringent conditions is meant that the nucleotide hybridises to filter-bound DNA or RNA in 3x sodium chloride/sodium citrate (SSC) at approximately 45 °C followed by at least one wash in 0.2x SSC/0.1% SDS at approximately 20-65 °C. Alternatively, a substantially similar peptide may differ by at least 1, but less than 3, 4, 5, 6, 7, 8, 9 or 10 amino acids from the sequences shown.

Due to the degeneracy of the genetic code, it is clear that any nucleic acid sequence described herein could be varied or changed without substantially affecting the sequence of the peptide, polypeptide or protein encoded thereby, to provide a functional variant thereof. Suitable nucleotide variants are those having a sequence altered by the substitution of different codons that encode the same amino acid within the sequence, thus producing a silent change. Other suitable variants are those having homologous nucleotide sequences but comprising all, or portions of, sequence, which are altered by the substitution of different codons that encode an amino acid with a side chain of similar biophysical properties to the amino acid it substitutes, to produce a conservative change. For example, small non-polar, hydrophobic amino acids include glycine, alanine, leucine, isoleucine, valine, proline, and methionine. Large non-polar, hydrophobic amino acids include phenylalanine, tryptophan and tyrosine. The polar neutral amino acids include serine, threonine, cysteine, asparagine and glutamine. The positively charged (basic) amino acids include lysine, arginine and histidine. The negatively charged (acidic) amino acids include aspartic acid and glutamic acid. It will therefore be appreciated which amino acids may be replaced with an amino acid having similar biophysical properties, and the skilled technician will know the nucleotide sequences encoding these amino acids.

According to one aspect of the invention, there is provided a monoclonal antibody that is an antagonist of the PD-12/PD-1 interaction.

Preferably, the antibody is an antibody or an antigen-binding fragment thereof that specifically binds to PD-L2 and/or PD-1 and interferes with the interaction between these two proteins. Even more preferably, the antibody is an antibody or an antigen-binding fragment thereof that specifically binds to PD-L2 and blocks its binding to its receptor PD-1.

Until now, there were no methods available to detect damaged and/or senescent cell *in vitro* or *in vivo.* As a result of the inventors' observation that damaged and/or senescent cells upregulate their expression of PD-L2, it is now possible, for the first time, to be able to identify the aforementioned cells *in vitro* or *in vivo* using a biomolecule that binds to PD-L2 and/or by analyzing mRNA levels. This in turn facilitates the diagnosis of chronic diseases and conditions that result from the accumulation of such cells, such as, for example, those referred to above.

Thus, in another aspect of the invention, there is provided a biomolecule that binds to PD-L2 or to PD-L2 mRNA for use in identifying damaged and/or senescent cells. In yet another aspect of the invention, there is provided a method of identifying damaged and/or senescent cells, the method comprising administering a biomolecule that binds to of PD-L2 or PD-L2 mRNA and detecting whether the agent has bound to PD-L2 or PD-L2 mRNA.

All of the features described herein (including any accompanying claim, abstract and drawing), and/or all of the steps of any method or process so disclosed, may be combined with any of the above aspects in any combination, except combinations where at least some of the features and/or steps are mutually exclusive.

For a better understanding of the invention, and to show how embodiments of the same may be carried into effect, reference will now be made, by way of example, to the Examples set forth below and the accompanying diagrammatic drawings.

### EXAMPLES

The invention will now be described by way of illustration only in the following examples.

As described in the Examples, the inventors have consistently observed the up-regulation of PD-L2 in damaged and/or senescence cell found in a variety of primary and tumor cell lines as well as in *in vivo* models of senescence. These observations imply that PD-L2 mRNA or protein levels could be used as biomarkers for the identification of damaged and/or senescent cells. Moreover, these observations strongly suggest that damaged and/or senescent cells may evade the immune system by inducing the expression of PD-L2, thus inhibiting immune cell activation via PD-1 receptors. Consequently, they have advantageously been able to develop a method whereby antagonists of the PD-L2/PD-1 interaction are able to eliminate damaged and/or senescent cells and can be used to treat, ameliorate or prevent a disease characterized by the presence of such cells.

The materials and methods employed in the studies described in the Examples were as follows, unless where otherwise indicated:

### Materials

### Cell lines

SK-Mel103 (human melanoma) and C2C12 (mouse myoblasts) cells were obtained from American Type Culture Collection.

HcMel3 and HcMel12 (mouse melanoma metastatic cells from B57BL6 mice) described in (Landsberg et al., 2012) were provided by Dr. Thomas Tüting (University of Bonn, Germany).

UT-SCC-38 and UT-SCC42-B cell lines (human HNSCC cells derived from primary lesions) were provided by Dr. Reidar Grenman (University of Turku, Finland) and have been previously described (Grénman et al., 1991; Pekkola-Heino et al., 1994, 1995).

HcMel3 and HcMel12 cells were maintained in RPMI (Gibco). The rest of the cell lines were maintained in DMEM (Gibco). All media were supplemented with 10% fetal bovine serum (Gibco) with antimicotic-antibiotic (Gibco), and incubated in 20% O₂ sand 5% CO₂ at 37 °C.

### Antibodies

The anti-53BP1 antibody was purchased to Abcam (ab172580), anti-PD-L2 (D7U8C^{™}) antibody was obtained from Cell Signaling (cat# 82723), anti-SMC1 antibody was from Bethyl laboratories (A300-056A) and Ki67 antibody from Master Diagnostica (0003110QD).

### Chemicals

Palbociclib (PD 0332991) was purchased to Selleckchem (#S1116); Nutlin was obtained from Sigma (NG287); Doxorubicin was purchased to Pharmacia and Upjohn. Bleomycin was obtained from Sigma (B8416). Rapamycin, rotenone and metformin were obtained from Sigma (53123-88-9, 83-79-4 and 1115-70-4 respectively); IKK inhibitor (BAY 11-7082) was obtained from Selleck Chemicals (cat# S2913); JAK2 inhibitor (NVP-BSK805) was from ApexBio (cat# A3675) and PIM inhibitor (ETP-47995) was synthesized in the CNIO. Mouse TLR agonists were purchased from InvivoGen (Mouse TLRI-9 agonist kit).

### SA-β gal assay

SA-*β* gal activity in cell lines, in tumor sections, xenografts and whole lungs, was assessed with the β-Galactosidase Staining Kit from Cell Signaling (#9860).

### Quautitative real-time PCR (qRT-PCR)

Total RNA was isolated with TRI Reagent (Sigma-Aldrich). The cDNA synthesis was performed with the "iScript Advanced cDNA Synthesis kit" from BIO-RAD (#1725037). The quantitative real-time PCR reaction was carried out with "GoTaq qPCR Master Mix" (Promega #A6002) in a 7500 Fast Real-Time PCR System (Applied Biosystems) using "MicroAmp Fast Optical 96-well reaction plate with barcode" (Thermo Fisher #4346906) and "MicroAmp Optical adhesive film" (Thermo Fisher #4311971).

Primer sequences are depicted below:

### Human qRT-PCR primers:

β-actin forward (SEQ ID NO:5): 5'-CAAGGCCAACCGCGAGAAGAT-3
β-actin reverse (SEQ ID NO:6): 5'-CCAGAGGCGTACAGGGATAGCAC-3'
PD-L1 forward (SEQ ID NO:7): 5'-TGGCATTTGCTGAACGCATTT-3'
PD-L1 reverse (SEQ ID NO:8): 5'-TGCAGCCAGGTCTAATTGTTTT-3'
PD-L2 forward (SEQ ID NO:9): 5'-ATTGCAGCTTCACCAGATAGC-3'
PD-L2 reverse (SEQ ID NO:10): 5'-AAAGTTGCATTCCAGGGTCAC-3'
TBP forward (SEQ ID NO:11): 5'-ATCAGTGCCGTGGTTCGT-3
TBP reverse (SEQ ID NO:12): 5'-TTCGGAGAGTTCTGGGATTG-3'

### Mouse qRT-PCR primers:

Actin forward (SEQ ID NO:13): 5'-GGCACCACACCTTCTACAATG-3
Actin reverse (SEQ ID NO:14): 5'-GTGGTGGTGAAGCTGTAGCC-3'
GAPDH forward (SEQ ID NO:15): 5'-TTCACCACCATGGAGAAGGC-3
GAPDH reverse (SEQ ID NO:16): 5'-CCCTTTTGGCTCCACCCT-3'
PD-L1 forward (SEQ ID NO:17): 5'- AGTCAATGCCCCATACCGC -3'
PD-L1 reverse (SEQ ID NO:18): 5'- TTCTGGATAACCCTCGGCCT -3'
PD-L2 forward (SEQ ID NO:19): 5'- CAAGCCTCAGCCTAGCAGAA -3'
PD-L2 reverse (SEQ ID NO:20): 5'- TCCATCCGACTCAGAGGGTC -3
IL-6 forward (SEQ ID NO:21): 5'-GTTCTCTGGGAAATCGTGGA -3'
IL-6 reverse (SEQ ID NO:22): 5'-GGTACTCCAGAAGACCAGAGGA -3'
p21 forward (SEQ ID NO:23): 5'- GTGGGTCTGACTCCAGCCC -3
p21 reverse (SEQ ID NO:24): 5'- CCTTCTCGTGAGACGCTTAC-3

### Methods

### SA-β gal assay

### Cell lines (Figs. 1A and 2A)

SK-Mel-103, UT-SCC-38 and UT-SCC-42B the cell lines were maintained in DMEM (Gibco) supplemented with 10% fetal bovine serum (Gibco) with antimycotic-antibiotic (Gibco), and incubated in 20% O₂ sand 5% CO₂ at 37 °C. SK-Mel-103 and UT-SCC-42B cells were split 1:10 every 3-4 days. UT-SCC-38 were split 1:3 every 3-4 days.

Eight days before the assay 20.000 SK-Mel-103 cells and 40.000 UT-SCC-38 and UT-SCC-42B cells were seeded into 6 well tissue culture plates (Falcon). The next day cells were treated with 2 µM Palbociclib. SK-Mel-103 cells were also treated with 10 nM Doxorubicin, 1 µM Nutlin or 6x10⁻³ units/ml Bleomycin. All the cells were incubated in the presence of the senescence-inducing agents for at least 7 days without changing the media. Control cells were seeded in 6 well plates 24-48 hours prior the SA-*β* gal assay.

SA-*β* galactosidase activity was assessed with the β-Galactosidase Staining Kit from Cell Signaling (#9860) following the manufactured instructions with one single variation: the pH of the "Staining Solution" (supplemented with buffers A and B and X-gal) was carefully adjusted to 6.

### Tissue sections (Figs. 4A and 6B)

To assess SA-*β* galactosidase activity of fibrotic and control kidneys (Fig. 4A), kidneys were embedded in optimal cutting temperature (OCT) compound (Leica Biosystems) and subsequently used for cryosections. SA-*β* galactosidase staining was performed using the Senescence β-Galactosidase Staining Kit (Cell Signaling #9860) according to manufacturer's instructions. Briefly, sections were fixed 10 min with a solution containing 2 % formaldehyde and 0.2 % glutaraldehyde in PBS, washed three times with PBS and then incubated with X-gal solution (adjusted to pH 6.0) for 4 hours at 37 °C. Slides were subsequently stained with nuclear fast red.

Embedded cryosections of control and palbociclib treated tumors (Fig. 6B) were stained using the Senescence β-Galactosidase Staining Kit (Cell Signaling #9860). Tumors were fixed with a solution containing 2 % formaldehyde and 0.2 % glutaraldehyde in PBS for 10 minutes. Next, samples were washed three times with PBS and stained with X-gal staining solution (adjusted to pH 6.0) over night (16 hours).

### Whole organs/xenografts (Figs. 5A and 6B)

SA-*β* galactosidase activity was assessed in whole mount lungs (Fig. 5A) and xenografts (Fig. 6B) using the Senescence β-Galactosidase Staining Kit (Cell Signaling #9860). Lungs were fixed for 45 minutes with a solution containing 2 % formaldehyde and 0.2 % glutaraldehyde in PBS for 45 minutes and washed three times with PBS. Fixed tissues were subsequently stained overnight (16 hours) with X-gal staining solution (adjusted to pH 6.0).

### Quautitative real-time PCR

### Cell extracts (Fig. 1B, Fig. 2C, Fig. 3 and Fig. 9)

SK-Mel-103, UT-SCC-38 and UT-SCC-42B (Figs. 1B and 2C) were seeded in 10 cm dishes and incubated with the indicated senescence-inducing agents at the following concentrations: 2 µM Palbociclib, 10 nM Doxorrubicin, 1 µM Nutlin and 6x10⁻³ units/ml Bleomycin. Treated cells were not further split and their media was not changed for the duration of the treatment. Control cells were split every 3-4 days as previously described. One week after the start of the treatment, total RNA was isolated from control and treated cells with TRI Reagent (Sigma-Aldrich #T9424) following the manufacturer instructions.

C2C12, HcMel3 and HcMel12 (Fig. 3) were treated with 12x10⁻³ units/ml bleomycin or doxorubicin 500 nM (C2C12) or 167 nM (HcMel3 and HcMe112) for 48 hours. Total RNA was isolated using TRI Reagent (Sigma-Aldrich #T9424) according to the manufacturer's instructions.

C2C12 cells (Fig. 9) were treated with 500 nM doxorubicin for 48 hours in the presence or absence of the following inhibitors at the indicated concentrations: IKKi (50 µM), PIMi (1 µM), rotenone (1 µM), JAKi (3 µM), rapamycin (50 nM) and metformin (5 mM) or cultured in pyruvate-depleted media (Fig. 9A). In addition, C2C12 cells were incubated for 6 hours in the presence or absence of TLRs agonists (Mouse TLR1-9 Agonist kit. Invivogen): TLR1/2 agonist (Pam3CSK4, 300 ng/ml), TLR2 agonist (HKLM, 10⁸ cells/ml), TLR3 agonist (Poly (I:C) HMW and Poly (I:C) LMW, 10 µg/ml), TLR4 agonist (LPS-EK, 10 µg/ml), TRL5 agonist (FLA-ST, 1 µg/ml), TRL6/2 agonist (FSL-1, 100 ng/ml), TRL7 agonist (ssRNA40, 5 µg/ml) and TLR9 agonist (ODN1826, 5 µM). Total RNA was isolated using TRI Reagent (Sigma-Aldrich #T9424) according to the manufacturer's instructions.

The isolated RNA was quantified using a nanodrop machine NanoVue (GE Healthcare). cDNA synthesis was performed with 2-7 µg of total RNA using the "iScript Advanced cDNA Synthesis kit" from BIO-RAD (#1725037). Quantitative real-time PCR was carried out with diluted cDNA (1:10) and "GoTaq qPCR Master Mix" (Promega A6002). Reactions were performed in triplicate in a 15 µl volume reaction mix prepared as follow: 7.5 µl 2X GoTaq qPCR Master Mix; 0.6 µl of pre-mixed primers (10 µM forward primer and 10 µM reverse primer); 1.9 µl nuclease-free water and 5 µl template cDNA (previously diluted 1:10).

The qRT-PCR reaction was performed on a 7500 Fast Real-Time PCR System (Applied Biosystems) using "MicroAmp Fast Optical 96-well reaction plate with barcode" (Thermo Fisher #4346906) and "MicroAmp Optical adhesive film" (Thermo Fisher #4311971). The reaction consisted in two holding stages (50 °C for 2:00 min and 95°C for 10:00 min) and 40 amplification reactions (95 °C for 00:15 min/ 60°C for 01:00 min/ 72 °C for 00:30 min) followed by a Melt Curve Stage.

The Ct values of the genes of interest were averaged and normalized to the housekeeping gene *β-actin.* For each cell line and condition at least two independent experiments were performed.

### Kidneys (Figs. 4B and 4C)

To isolate total RNA (Fig. 4B), kidney biopsies were lysed in TRI Reagent (Sigma-Aldrich #T9424) according to manufacturer's instructions. cDNA was synthesized using 2 µg of total RNA. cDNA synthesis and RT-qPCR were performed as previously described. The Ct values of the genes of interest were averaged and normalized to the housekeeping gene *β-actin.*

### Lungs (Fig. 5B)

For gene expression analysis lungs were isolated and embedded in optimal cutting temperature (OCT) compound (Leica Biosystems). To obtain total RNA of OCT embedded tissue samples, 10 cryostat sections of 10 µm thickness were cut and excess OCT were removed. Cryosections were lysed in TRI Reagent (Sigma-Aldrich #T9424), pooled and processed according to manufacturer's instructions. cDNA was synthesized using 2 µg of total RNA. cDNA synthesis and RT-qPCR were performed as previously described. The Ct values of the genes of interest were averaged and normalized to the housekeeping gene *Gapdh.*

### Xenografts (Fig. 7A)

Tumours were isolated and embedded in optimal cutting temperature (OCT) compound (Leica Biosystems). To obtain total RNA 10 cryosections of 10 µm thickness were cut and the excess OCT was removed. Cryosections were then pooled and lysed in TRI Reagent (Sigma-Aldrich #T9424) for total RNA and processed according to manufacturer's instructions. Two µg of total RNA was used to synthesize cDNA and RT-qPCR was performed as previously described. Human specific primers for PD-L1 and PD-L2 were used. The Ct values of the genes of interest were averaged and normalized to the housekeeping gene *TPB.*

### Aged animals (Fig. 8)

Liver samples from young mice (10 weeks of age) and old mice (75 weeks of age) were collected and embedded in optimal cutting temperature (OCT) compound (Leica Biosystems). To obtain total RNA of OCT embedded tissue samples, 10 cryostat sections of 10 µm thickness were cut and excess OCT were removed. Cryosections were lysed in TRI Reagent (Sigma-Aldrich #T9424), pooled and processed according to manufacturer's instructions. cDNA was synthesized using 2 µg of total RNA. cDNA synthesis and RT-qPCR were performed as previously described. The Ct values of the genes of interest were averaged and normalized to the housekeeping *gene β-actin.*

### Western blotting (Fig. 7B)

Tumours were isolated and embedded in optimal cutting temperature (OCT) compound (Leica Biosystems). To obtain protein extracts, 10 cryosections of 10 µm thickness were cut and the excess OCT was removed. Cryosections were then pooled and lysed in RIPA lysis buffer (150 mM sodium chloride, 1.0% NP-40, 0.5% sodium deoxycholate, 0.1% SDS, 50 mM Tris, pH 8.0) containing protease inhibitors for protein lysates. Samples were sonicated 5 minutes (30 sec. on/off), resuspended in sample buffer and denatured for 5 minutes at 95 °C. Equal amounts of protein lysates were separated on 4-12% NuPAGE gels (Invitrogen). Proteins were transferred to nitrocellulose membrane (GE Healthcare) and membranes were incubated with the anti-PD-L2 (1:1000) (D7U8C™; Cell Signaling cat# 82723) or anti-SMC1 (1:1000) (Bethyl laboratories, A300-056A).

Followed by detection with horseradish peroxidase-conjugated secondary antibodies (1:1000) (Rockland). Bound proteins were visualized with ECL (GE Healthcare) and chemiluminiscence was detected with a Molecular Imager Gel Dox XR System (Bio-Rad).

### Immunofluorescence (Fig. 2B)

SK-Mel-103 cells were seeded at 50% confluency on round 12 mm diameter coverslips (Marienfeld #0111520) previously placed inside 24 well plates. The following day, cells were treated with the senescence-inducing agents: 2 µM Palbociclib, 10 nM, 1 µM Nutlin and 6x10⁻³ units/ml Bleomycin. Treated cells were not further split and their media was not changed for the duration of the treatment (7 days). Control cells were seeded similarly to treated, cells 24-48 hours prior the beginning of the procedure. Seven days after addition of the senescence-inducing agents, control and treated cells were washed with 1X PBS and Fixed on 2% paraformaldehyde-PBS for 10 minutes. The cells were then permeabilized with 0.25% Triton-PBS for 5 minutes, washed twice with 1x PBS and blocked with 5% BSA-PBS for 30 minutes. Next, the cells were incubated for 30 minutes with anti-53BP1 antibody (Abcam #ab172580) previously diluted 1:100 in blocking buffer (5% BSA-PBS), washed 3 times for 10 minutes with 1X PBS and incubated in the dark for 30 additional minutes with the secondary antibody: goat anti-rabbit Alexa Fluor 546 (ThermoFisher #A-11010) diluted 1:400 in blocking buffer. Finally, the cells were washed again 3 times for 10 minutes with 1X PBS and incubated with DAPI (Sigma # D9542) 300 nM in 1X PBS for 10 minutes. All the incubations and washed were performed at room temperature in 24 well plates. Once the immunostaining procedure was finished, the coverslips were carefully removed from the 24 well plates and placed outside down on glass slides (Menzel-Glaser #0111520) over a drop of ProLong Gold anti-fade reagent (Invitrogen # P36930). The glass slides were stored in the dark at room temperature for at least 24 hours to allow the anti-fade to dry and subsequently images of the immunostained nuclei were acquired with a SP5-MP Confocal microscope (Leica).

### Immunohistochemistry (Fig. 6B)

For immunohistochemistry analysis, tumours were fixed in formalin and embedded in paraffin blocks. Sections were then stained with Ki67 following standard procedures.

### Animal procedures

### Unilateral ureteral obstruction (UUO) (Fig. 4)

Kidney fibrosis was induced in C57BL6 mice (ENVIGO) by the surgical obstruction of the left ureteral conduct (UUO). Briefly, mice were anesthetized and surgical incisions were made to access the abdominal cavity. Subsequently, two ligations of the left ureter were made with a 6/0 silk (LA BOUVET) close to the renal hilum. Next, the peritoneum was sutured with 3/0 silk (B. Braun Aesculap) and the skin with surgical staples. Non-operated (control) mice and mice with ligated kidneys (fibrotic) were sacrificed 10 days (Fig. 4A) or 12-13 days (Fig. 4B) post-ligation, to analyze SA-*β* galactosidase activity and gene expression respectively.

### Lung fibrosis (Fig. 5)

Lung fibrosis was induced in C57BL6 mice (ENVIGO) by intratracheal delivery of a single dose of bleomycin (1.5U/kg) or vehicle (PBS). 14 days after intratracheal injection, lung samples of vehicle (control) or bleomycin (fibrotic)-treated mice were collected for the analysis of SA-*β* gal activity and gene expression.

### Xenografts (Figs. 6 and 7)

SK-Mel-103 cells (1x10⁶) were subcutaneously inoculated in immunodeficient nu/nu mice (ENVIGO). When tumors reached an average size of 200 mm³, mice were treated with vehicle (50 mM sodium lactate, pH 4.0) or palbociclib 100 mg/kg by oral gavage on week days for up to 10 days. Tumour progression was monitored by caliper measurement. Tumor volume calculated using the formula Volume = (width)² x length/2. Mice were sacrificed and samples collected when control individuals reached the human endpoint (Fig. 6A) or 5 days post palbociclib treatment (Fig. 6B) to obtain tumors of comparable sizes (Fig. 6B) which were subsequently used for gene (Fig. 7A) and protein expression analysis (Fig. 7B).

All the animals used in these experiments, unless specified differently, were males mice of 10-16 weeks of age. Animal experimentation at the CNIO was performed according to protocols approved by the CNIO-ISCIII Ethics Committee for Research and Animal Welfare (CEIyBA).

### Results

### Example 1: Evidence in cultured cells that PD-L2 is highly and preferentially expressed in damaged and or senescent cells

The inventors have observed that prolonged treatment of tumor cell lines with palbociclib, a CDK4/CDK6 specific inhibitor developed by Pfizer, induced phenotypic changes consistent with a senescent response (increased size and flat morphology, cell cycle arrest and increased SA-*β* gal activity) in a variety of human tumor cells such as the melanoma SK-Mel-103 and head and neck squamous cell carcinoma (HNSCC) SCC38 and SCC42B cell lines (Figs. 1A and 2A). Under these conditions, senescent cells showed up-regulation of PD-L2 but not PD-L1 mRNA levels (Figs. 1B and 2C). In addition to palbociclib, the genotoxic drug bleomycin and the Mdm2 inhibitor nutlin, induce senescence in the SK-Mel-103 cells (Fig. 2A) and had a similar effect on PD-L2 mRNA levels (Fig. 2C). Since neither palbociclib nor nutlin caused DNA damage in these cells, as evidenced by the absence of 53BP1 foci in the chromatin (Fig. 2B), the inventors were able to conclude that, in this case as well, PD-L2 induction is associated with a senescent response rather than a DNA damage response.

Next, the inventors extended their observations to mouse cell lines. They observed PD-L2 mRNA up-regulation in the mouse melanoma cell lines B16F1 and B16F10 upon treatment with palbociclib or bleomycin (data not shown). In addition, they found that short time (48 hours) treatment with the genotoxic drugs bleomycin or doxorubicin resulted in a dramatic increase in PD-L2 mRNA in the mouse myoblast cell line C2C12 (Fig. 3A) and the mouse melanoma cell lines HcMel3 and HcMel13 (Fig. 3B and 3C). Since the manifestation of phenotypic changes associated with senescence requires longer periods of time to develop, this observation suggest that PD-L2 can be induced in response to the implementation of DNA-damage response in addition to a senescent response. However, this distinction maybe futile since, as mentioned above, cellular senescence causes persistent DNA damage and senescent-associated markers may cause DNA damage even in the absence of cell cycle arrest. Whether PD-L2 induction is associated with an early senescent response or it is caused by the implementation of two different cellular programs, both triggered by DNA damage, will require further clarification.

### Example 2: Evidence in preclinical models of lung and kidney fibrosis that PD-L2 is highly and preferentially expressed in damaged and or senescent cells

Fibrotic pathologies, including lung and kidney fibrosis have been recently linked to the occurrence of senescence. The inventors used mice models of these diseases to study the regulation of PD-L2 *in vivo.* Lung fibrosis was induced in mice by intratracheal delivery of a single dose of bleomycin. Kidney fibrosis was caused by the surgical obstruction of the ureteral conduct. Both lung and kidney fibrosis resulted in the accumulation of senescent cells as revealed by the detection of SA-*β* galactosidase positive staining in tissue samples from fibrotic kidneys (Figs. 4A) and in whole fibrotic lungs (Fig. 5A). In the model of kidney fibrosis, this was reinforced by the transcriptional up-regulation of two well-characterized senescent markers: p21 and IL-6, in fibrotic organs (Fig. 4B). In agreement with the inventors' previous observations, they found a significant and specific up-regulation of PD-L2 mRNA, but not of PD-L1 mRNA, in fibrotic organs, thereby confirming that PD-L2 is induced in association with cellular senescence (Figs. 4C and 5B).

### Example 3: Evidence in preclinical models of chemotherapy that PD-L2 is highly and preferentially expressed in damaged and or senescent cells

*In vivo* treatment with palbociclib, delivered by oral gavage, was shown to halt the growth of mouse xenografts of the human melanoma cell line SK-Mel-103 (Fig. 6A). This CDK4/CDK6 inhibitor induced a strong senescent response as evidenced by the intense staining of whole tumors and tumor sections (Fig. 6B). Increased SA-*β* galactosidase activity strongly correlated with the absence of Ki67 positive proliferative cells (Fig. 6B). When SK-Mel-103 xenografts were analyzed for PD-L1 and PD-L2 expression, the inventors observed that PD-L2 mRNA was selectively up-regulated in xenografts from palbociclib-treated mice (Fig. 7A). Accordingly, PD-L2 protein levels are also increased in senescent tumors (Fig. 7B).

### Example 4: Evidence in animal models that PD-L2 is upregulated in aged individuals

The inventors have observed that tissues (including liver) from old mice (75 weeks of age) have statistically significant higher levels of PD-L2 mRNA compared to equivalent samples from young mice (10 weeks of age) (Fig. 8). This demonstrates in a physiological setting that PD-L2 levels are associated to the accumulation of senescent cells that is characteristic of aging.

Taken together these observations demonstrate that PD-L2 is induced in response to DNA-damage and senescent-inducing stimuli.

### Example 5: Evidence of the molecular mechanism responsible for PD-L2 up-regulation in damaged/senescent cells

The inventors prevented PD-L2 induction *in vitro* using commercial inhibitors against enzymes known to be involved in senescence/DNA-damage responses, such as rapamycin, rotenone, metformin, and JAK and NFkB inhibitors (Fig. 9A). They also used agonists of TLRs that activate NFkB and may mediate the autocrine and paracrine effects of the SASP (Fig. 9B). This experimental approach gave the inventors a first insight into the mechanism responsible for the transcriptional induction of PD-L2. To further characterize the cellular pathway that signals PD-L2 up-regulation, the inventors will implement appropriate knock-down/activating strategies.

### Example 6: Evidence that the expression of PD-L2 ligand by damaged and/or senescent cells interferes with T cell activation in vitro

The inventors observe the activation of human T cells (Jurkat) in the presence of control/senescent human tumor cells. Jurkat cells can be activated *in vitro* by the combination of 12-O-Tetradecanoylphorbol-13-acetate (TPA), that activates the enzyme protein kinase C, and phytohemagglutinin (PHA), a lectin that binds glycosylated proteins on the surface of immune cells. Activated T cells strongly induce IL-2; thus by analyzing IL-2 mRNA levels or by immunodetection of secreted IL-2 by ELISA, the inventors can infer the effect of co-cultured control and senescent cells in T lymphocyte activation. The inventors observe that PD-L2 up-regulation interferes with T cell activation and thus reduces IL2 induction. In addition, the inventors observe that the inhibitory effect of senescent cells is reversed by the presence of blocking anti-PD-1 or anti-PD-L2, but not of anti-PD-L1, antibodies. Finally, the inventors observe from knocking-down PD-L2 using lentiviral shRNA plasmids that PD-L2 is directly involved in the inhibition of T cell activation.

### Example 7: Evidence that interference with PD-L2/PD-1 interaction increases T cell activation and promotes the immunoclearance of damaged/senescent cells in vivo.

The inventors have proben the up-regulation of PD-L2 in three *in vivo* models of senescence: bleomycin-induced lung fibrosis (Fig. 4), unilateral ureteral obstruction (UUO)-induced renal fibrosis (Figs. 5) and organismal aging (Fig. 8). To demonstrate the functional implications of this effect, fibrotic mice are treated with blocking anti-PD-1 and anti-PD-L2 antibodies and the progression of these diseases analyzed. By preventing PD-1/PD-L2 interaction, these antibodies potentiate T cell immunity and improve the clearance of senescent cells by the immune system, facilitating the regression of the fibrotic areas. In addition, the effect of blocking PD-1/PD-L2 interaction on infiltrating T lymphocytes is analyzed in the senescent organs. Flow cytometry is used to analyze and characterize the number and activation status of infiltrating T cells.

The inventors have found that in mouse cell lines, DNA damage is a potent inducer of PD-L2 (Fig. 3). In this case a dramatic up-regulation of PD-L2 occurs before the onset of senescence. This effect is authenticated *in vivo* by generating xenografts with the mouse melanoma cell lines HcMel3 and HcMel 12. These cell lines are isogenic and can induce tumors in mice with a competent immune system. PD-L2 up-regulation follows the treatment of these xenografts with the genotoxic drugs doxorubicin or irradiation, and tumor progression in the presence of blocking anti-PD-1 or anti-PD-L2 antibodies is then analyzed. These therapeutic antibodies are shown to facilitate tumor regression. To test the direct involvement of PD-L2, the inventors knock-down PD-L2 in HcMel3 and HcMe112 cell lines. This prevents PD-L2 up-regulation by genotoxic damage and cancels the effect of anti PD-1 and/or PD-L2 antibodies.

### Conclusion

In conclusion, the present inventors have shown for the first time that PD-L2 is expressed not only on antigen-present cells and other immune cells, but also on damaged and/or senescent cells. This finding is of great importance as it provides a method for the identification of damaged and/or senescent cells that could be of prognostic value for those conditions associated to the presence of damaged and/or senescent cells and, even more importantly, provides a therapeutic strategy for the elimination of, and the treatment of diseases and conditions associated with, damaged and/or senescent cells.

### References

Almagro J.C. and Fransson J. Humanization of antibodies. Frontiers in Bioscience 13, 1619-1633 (2008).
Althubiti, M., Lezina, L., Carrera, S., Jukes-Jones, R., Giblett, S.M., Antonov, A., Barlev, N., Saldanha, G.S., Pritchard, C.A., Cain, K., et al. (2014). Characterization of novel markers of senescence and their prognostic potential in cancer. Cell Death Dis. 5, e1528.
Baker, D.J., Wijshake, T., Tchkonia, T., LeBrasseur, N.K., Childs, B.G., van de Sluis, B., Kirkland, J.L., and van Deursen, J.M. (2011). Clearance of p16Ink4a-positive senescent cells delays ageing-associated disorders. Nature 479, 232-236.
Baker, D.J., Childs, B.G., Durik, M., Wijers, M.E., Sieben, C.J., Zhong, J., A. Saltness, R., Jeganathan, K.B., Verzosa, G.C., Pezeshki, A., et al. (2016). Naturally occurring p16Ink4a-positive cells shorten healthy lifespan. Nature 530, 184-189.
Chang, J., Wang, Y., Shao, L., Laberge, R.-M., Demaria, M., Campisi, J., Janakiraman, K., Sharpless, N.E., Ding, S., Feng, W., et al. (2016). Clearance of senescent cells by ABT263 rejuvenates aged hematopoietic stem cells in mice. Nat. Med. 22, 78-83.
Chen, D., Xia, D., Pan, Z., Xu, D., Zhou, Y., Wu, Y., Cai, N., Tang, Q., Wang, C., Yan, M., et al. (2016). Metformin protects against apoptosis and senescence in nucleus pulposus cells and ameliorates disc degeneration in vivo. Cell Death Dis. 7, e2441.
Childs, B.G., Baker, D.J., Wijshake, T., Conover, C.A., Campisi, J., and van Deursen, J.M. (2016). Senescent intimal foam cells are deleterious at all stages of atherosclerosis. Science (80). 354, 472-477.
Childs, B.G., Durik, M., Baker, D.J., and van Deursen, J.M. (2015). Cellular senescence in aging and age-related disease: from mechanisms to therapy. Nature Medicine 21, 1424-1435. doi:10.1038/nm.4000
Cole, S.P.C. et al., in: Monoclonal Antibodies and Cancer Therapy, Alan R. Liss, Inc., pp. 77-96 (1985)
Coppe, J.-P., Patil, C.K., Rodier, F., Sun, Y., Muñoz, D.P., Goldstein, J., Nelson, P.S., Desprez, P.-Y., and Campisi, J. (2008). Senescence-associated secretory phenotypes reveal cell-nonautonomous functions of oncogenic RAS and the p53 tumor suppressor. PLoS Biol. 6, 2853-2868.
Cote, R. J. et al. Generation of human monoclonal antibodies reactive with cellular antigens. Proc Natl Acad Sci U S A 80(7), 2026-2030 (1983).
Ewald, J.A., Desotelle, J.A., Wilding, G., and Jarrard, D.F. (2010). Therapy-Induced Senescence in Cancer. JNCI J. Natl. Cancer Inst. 102, 1536-1546.
Fagarasan, S., and Honjo, T. (2000). T-Independent immune response: new aspects of B cell biology. Science 290, 89-92.
He, Y.F., Zhang, G.M., Wang, X.H. et al., (2004). "Blocking programmed death-1 ligand-PD-1 interactions by local gene therapy results in enhancement of antitumor effect of secondary lymphoid tissue chemokine," Journal of Immunology, 173(8),4919-4928.
Hobo, W., Mass, F., Adisty, N., deWitte, T., Schapp, N., van der Voort, R., Dolstra, H. (2010). siRNA silencing of PD-L1 and PD-L2 on dendritic cells augments expansion and function of minor histocompatibility antigen-specific CD8+ T cells. Blood 116:4501-4511; doi: https://doi.org/10.1182/blood-2010-04-278739
Kohler, G. & Milstein, C. Continuous cultures of fused cells secreting antibody of predefined specificity. Nature 256, 495-497 (1975).
Muñoz-Espin, D., and Serrano, M. (2014). Cellular senescence: from physiology to pathology. Nat. Rev. Mol. Cell Biol. 15, 482-496.
Naidoo, J., Page, D.B., Li, B.T., Connell, L.C., Schindler, K., Lacouture, M.E., Postow, M.A., and Wolchok, J.D. (2015). Toxicities of the anti-PD-1 and anti-PD-L1 immune checkpoint antibodies. Ann. Oncol. 26, mdv383.
Ness, K.K., Armstrong, G.T., Kundu, M., Wilson, C.L., Tchkonia, T., and Kirkland, J.L. (2015). Frailty in childhood cancer survivors. Cancer 121, 1540-1547.
Ohaegbulam, K.C., Assal, A., Lazar-Molnar, E., Yao, Y., and Zang, X. (2015). Human cancer immunotherapy with antibodies to the PD-1 and PD-L1 pathway. Trends Mol. Med. 21, 24-33.
Pardoll, D.M. (2012). The blockade of immune checkpoints in cancer immunotherapy. Nat. Rev. Cancer 12, 252-264.
Parekh, V.V., Lalani, S., Kim, S. et al. (2009). "PD-1/PD-L blockade prevents anergy induction and enhances the anti-tumor activities of glycolipid-activated invariant NKT cells," Journal of Immunology, vol. 182(5), 2816-2826.
Rayess, H., Wang, M.B., Srivatsan, E.S. Cellular senescence and tumor suppressor gene pl6. Int J Cancer. 2012 Apr 15; 130(8): 1715-1725.
Roos, C.M., Zhang, B., Palmer, A.K., Ogrodnik, M.B., Pirtskhalava, T., Thalji, N.M., Hagler, M., Jurk, D., Smith, L.A., Casaclang-Verzosa, G., et al. (2016). Chronic senolytic treatment alleviates established vasomotor dysfunction in aged or atherosclerotic mice. Aging Cell 15, 973-977.
Rozali, E.N., Hato, S. V, Robinson, B.W., Lake, R.A., and Lesterhuis, W.J. (2012). Programmed death ligand 2 in cancer-induced immune suppression. Clin. Dev. Immunol. 2012, 656340.
Ruhland, M.K., Loza, A.J., Capietto, A.-H., Luo, X., Knolhoff, B.L., Flanagan, K.C., Belt, B.A., Alspach, E., Leahy, K., Luo, J., et al. (2016). Stromal senescence establishes an immunosuppressive microenvironment that drives tumorigenesis. Nat. Commun. 7, 11762.
Sanoff, H.K., Deal, A.M., Krishnamurthy, J., Torrice, C., Dillon, P., Sorrentino, J., Ibrahim, J.G., Jolly, T.A., Williams, G., Carey, L.A., et al. (2014). Effect of cytotoxic chemotherapy on markers of molecular age in patients with breast cancer. J. Natl. Cancer Inst. 106, dju057.
Sharma, P., and Allison, J.P. (2015). Immune checkpoint targeting in cancer therapy: toward combination strategies with curative potential. Cell 161, 205-214.
Sorrentino, J.A., Krishnamurthy, J., Tilley, S., Alb, J.G., Burd, C.E., and Sharpless, N.E. (2014). p16INK4a reporter mice reveal age-promoting effects of environmental toxicants. J. Clin. Invest. 124, 169-173.
Sun, Y., Campisi, J., Higano, C., Beer, T.M., Porter, P., Coleman, I., True, L., and Nelson, P.S. (2012). Treatment-induced damage to the tumor microenvironment promotes prostate cancer therapy resistance through WNT16B. Nat. Med. 18, 1359-1368.
Thompson, J.D., Higgins, D.G. and Gibson, T.J. (1994). CLUSTAL W: improving the sensitivity of progressive multiple sequence alignment through sequence weighting, position-specific gap penalties and weight matrix choice. Nucleic Acids Res 22, 4673-4680.
Wood, W.A., Krishnamurthy, J., Mitin, N., Torrice, C., Parker, J.S., Snavely, A.C., Shea, T.C., Serody, J.S., and Sharpless, N.E. (2016). Chemotherapy and Stem Cell Transplantation Increase pl6INK4a Expression, a Biomarker of T-cell Aging. EBioMedicine 11, 227-238.
Xu, M., Palmer, A.K., Ding, H., Weivoda, M.M., Pirtskhalava, T., White, T.A., Sepe, A., Johnson, K.O., Stout, M.B., Giorgadze, N., et al. (2015). Targeting senescent cells enhances adipogenesis and metabolic function in old age. Elife 4, e12997.
Yosef, R., Pilpel, N., Tokarsky-Amiel, R., Biran, A., Ovadya, Y., Cohen, S., Vadai, E., Dassa, L., Shahar, E., Condiotti, R., et al. (2016a). Directed elimination of senescent cells by inhibition of BCL-W and BCL-XL. Nat. Commun. 7, 11190.
Yosef, R., Pilpel, N., Tokarsky-Amiel, R., Biran, A., Ovadya, Y., Cohen, S., Vadai, E., Dassa, L., Shahar, E., Condiotti, R., et al. (2016b). Directed elimination of senescent cells by inhibition of BCL-W and BCL-XL. Nat. Commun. 7, 11190.
Zacarias-Fluck, M.F., Morancho, B., Vicario, R., Luque Garcia, A., Escorihuela, M., Villanueva, J., Rubio, I.T., and Arribas, J. (2015). Effect of Cellular Senescence on the Growth of HER2-Positive Breast Cancers. JNCI J. Natl. Cancer Inst. 107, djv020-djv020.
Zhu, Y., Tchkonia, T., Pirtskhalava, T., Gower, A.C., Ding, H., Giorgadze, N., Palmer, A.K., Ikeno, Y., Hubbard, G.B., Lenburg, M., et al. (2015). The Achilles' heel of senescent cells: from transcriptome to senolytic drugs. Aging Cell 14, 644-658.
Zhu, Y., Tchkonia, T., Fuhrmann-Stroissnigg, H., Dai, H.M., Ling, Y.Y., Stout, M.B., Pirtskhalava, T., Giorgadze, N., Johnson, K.O., Giles, C.B., et al. (2016). Identification of a novel senolytic agent, navitoclax, targeting the Bcl-2 family of anti-apoptotic factors. Aging Cell 15, 428-435.
Ziegler, D. V., Wiley, C.D., and Velarde, M.C. (2015). Mitochondrial effectors of cellular senescence: beyond the free radical theory of aging. Aging Cell 14, 1-7.

## Claims

1. A method for the detection of the presence of damaged and/or senescent cells in an assayed sample, wherein the levels of a product (mRNA or protein) of the PD-L2 gene or a product of a gene operatively linked to the PD-L2 promoter are determined and the presence of damaged and/or senescent cells is acknowledged when the levels are higher than a reference value.

2. The method according to claim 1, wherein the levels of mRNA are determined by qRT-PCR.

3. The method according to claim 1, wherein the level of PD-L2 mRNA or protein is determined by contacting the sample with a molecule that binds to PD-L2 protein or PD-L2 mRNA, detecting whether the binding of the molecule to PD-L2 or PD-L2 mRNA and identifying as damaged or senescent cells those cells where the binding of the molecule to PD-L2 or PD-L2 mRNA can be detected or where the level of bound molecule is higher than a reference value.

4. A method for the identification of damaged and/or senescent cells in a sample, the method comprising administering a molecule that binds to PD-L2 protein or PD-L2 mRNA, detecting whether the binding of the molecule to PD-L2 or PD-L2 mRNA and identifying as damaged or senescent cells those cells where the binding of the molecule to PD-L2 or PD-L2 mRNA can be detected or where the level of bound molecule is higher than a reference value.

5. The method of any one of claims 1 to 4, wherein the sample is a biological sample that has been taken out from a subject, a culture of cells or a group of cells separated from the biological sample or the culture of cells.

6. A method for the detection of damaged and/or senescent cells as claimed in any one of claims 1 to 5, for use in the prognosis of a disease or condition **characterised by** the presence of damaged and/or senescent cells.

7. A method of eliminating damaged and/or senescent cells, the method comprising contacting the cells with an antagonist of the PD-L2/PD-1 interaction.

8. A method of eliminating damaged and/or senescent cells, the method comprising administering a therapeutically effective amount of an antagonist of the PD-L2/PD-1 interaction to a subject in need thereof.

9. A method of treating, ameliorating or preventing a disease or condition **characterised by** the presence of damaged and/or senescent cells, the method comprising administering to a subject in need thereof such treatment, a therapeutically effective amount of an antagonist of the PD-L2/PD-1 interaction.

10. An antagonist of PD-L2/PD-1 interaction for use in eliminating damaged/or senescent cells in a subject.

11. An antagonist of PD-L2/PD-1 interaction for use in treating, ameliorating or preventing a disease associated to the presence of damaged and/or senescent cells.

12. An antagonist according to either claim 10 or claim 11, wherein the antagonist comprises a protein, peptide, antibody, chemical compound, genetic material, small molecule, drug or RNAi reagent.

13. An antagonist according to claim 12, wherein the antibody is an antibody that specifically binds PD-L2 and/or PD-1, or antigen-binding fragment thereof.

14. An antagonist according to claim 13, wherein the antibody is an anti-PD-L2 antibody, or antigen binding fragment thereof.

15. The method according to any one of claims 7 to 9 or an antagonist of PD-L2/PD-1 interaction for use according to claim 10 or 11, wherein the antagonist is a molecule that specifically binds to PD-L2 and/or PD-1 and blocks the ability of PD-L2 to interact with or bind to PD-1.

16. The method according to any one of claims 7 to 9 or an antagonist of PD-L2/PD-1 interaction for use according to claim 10 or 11, wherein the antagonist is a molecule capable to inhibit, diminish or supress PD-1 and/or PD-L2 expression.

17. The method according to claim 8 or the antagonist of PD-L2/PD-1 interaction for use according to claim 11 for use in treating a disease or condition which is selected from: a degenerative disease; type 2 diabetes; sarcopenia; cataracts, radiation-induced oral mucositis; brain and heart aneurysms; glaucoma; Alzheimer's disease, Parkinson's disease; cystic fibrosis, intestinal bowl disease osteoarthritis and inverted disc degeneration, a fibrotic disease, including systemic sclerosis, pulmonary fibrosis, idiopathic pulmonary fibrosis, kidney fibrosis, liver fibrosis, heart fibrosis, oral mucous fibrosis, pancreatic fibrosis atherosclerosis, brain glial scar, benign lesions such as nevi and skin scars post-wound healing; Wiedemann-Rautenstrauch syndrome of neonatal progeria, the Werner syndrome of adult progeria, Hutchinson-Gilford syndrome, Rothmund Thompson syndrome, Mulvill-Smith syndrome, Cockayne syndrome, Dyskeratosis Congenita; cancer, aplastic anaemia, emphysema, degeneration of cartilage, obesity, macular degeneration, glaucoma, pulmonary hypertension, chronic obstructive pulmonary disease, renal transplantation or aging.

18. An antagonist of PD-L2/PD-1 interaction for use according to claim 10, wherein the use is in combination with chemotherapy, anti-retroviral drugs, corticoids, PPAR gamma agonists and/or exposure to medical radiation or non-medical radiation.

19. An antagonist of PD-L2/PD-1 interaction for use according to claim 18, for use in increasing the therapeutic effect of chemotherapy or radiotherapy.

20. A damaged and/or senescent cells-treatment composition comprising an antagonist of the PD-L2/PD-1 interaction and a pharmaceutically acceptable vehicle.

21. A process for making the damaged and/or senescent cells-treatment composition according to claim 20, the process comprising contacting a therapeutically acceptable amount of an antagonist of the PD-L2/PD-1 interaction and a pharmaceutically acceptable vehicle.

22. A monoclonal antibody that is an antagonist of the PD-L2/PD-1 interaction.

23. A molecule that binds to PD-L2 or PD-L2 mRNA for use in identifying damaged and/or senescent cells.

24. A molecule according to claim 23, or a method according to any one of claims 3 to 6, wherein the molecule comprises a protein, peptide, antibody, chemical compound, genetic material, small molecule, drug or RNAi reagent.

25. A molecule or method according to claim 24, wherein the antibody is an antibody that specifically binds PD-L2, or antigen binding fragment thereof.

26. A molecule according to any of claims 23 to 25, or a method according to any of claims 3 to 6, 24 or 25, wherein the molecule comprises a contrast material that is visible using Magnetic Resonance Imaging (MRI), Computed Tomography (CT) and/or Optical Imaging (OI).
